# FASCICULE DE BREVET EUROPEEN

(11) **EP 3 386 510 B1**
(45) Date de publication et mention de la délivrance du brevet: **31.01.2024**
(21) Numéro de dépôt: 16825807.7
(22) Date de dépôt: 09.12.2016
(51) Int. Cl.: A61K 31/496, A61K 31/706, A61K 31/7064, A61K 31/7076, A61K 31/7084, A61P 21/00

(54) **DERIVES PHOSPHORES POUR LA PREVENTION OU LE TRAITEMENT DES MYOPATHIES ET TRAUMATISMES MUSCULAIRES**
PHOSPHORDERIVATE ZUR VORBEUGUNG ODER BEHANDLUNG VON MUSKULÄREN MYOPATHIEN UND TRAUMATISCHEN MUSKELVERLETZUNGEN
PHOSPHOROUS DERIVATIVES FOR THE PREVENTION OR TREATMENT OF MUSCULAR MYOPATHIES AND TRAUMATIC INJURIES TO MUSCLES

(30) Priorité: 09.12.2015 FR 1562040
(43) Date de publication de la demande: 17.10.2018
(73) Titulaire: Centre National de la Recherche Scientifique, 75016 Paris (FR)
(72) Inventeur: CANCELA, José-Manuel, 91120 Palaiseau (FR); DE LA PORTE, Sabine, 78000 Versailles (FR); DE ZELICOURT, Antoine, 78000 Versailles (FR)
(74) Mandataire: Novagraaf Technologies
(86) Numéro de dépôt international: PCT/FR2016/053289
(87) Numéro de publication internationale: WO 2017/098173

(56) Documents cités:
- WO-A1-2009/101399
- WO-A2-2011/003018
- IT-A1- FI20 070 077
- US-A1- 2009 306 006
- US-A1- 2012 308 542
- MICHELLE F. GOODY ET AL: "NAD+ Biosynthesis Ameliorates a Zebrafish Model of Muscular Dystrophy", PLOS BIOLOGY, vol. 10, no. 10, 23 octobre 2012 (2012-10-23), page e1001409, XP055291295, DOI: 10.1371/journal.pbio.1001409
- DATABASE CA [Online] CHEMICAL ABSTRACTS SERVICE, COLUMBUS, OHIO, US; QU, HONGYAN ET AL: "Protective effect of nicotinamide adenine dinucleotide on adriamycin-induced cardiomyopathy in rats", XP002760361, extrait de STN Database accession no. 2008:1345956
- ANNA KA YEE KWONG ET AL: "Catalysis-Based Inhibitors of the Calcium Signaling Function of CD38", BIOCHEMISTRY, vol. 51, no. 1, 10 janvier 2012 (2012-01-10), pages 555-564, XP055078673, ISSN: 0006-2960, DOI: 10.1021/bi201509f
- HONG JIANG ET AL: "Mechanism-Based Small Molecule Probes for Labeling CD38 on Live Cells", JOURNAL OF THE AMERICAN CHEMICAL SOCIETY, vol. 131, no. 5, 11 février 2009 (2009-02-11), pages 1658-1659, XP055291340, US ISSN: 0002-7863, DOI: 10.1021/ja808387g & HONG JIANG: "Supporting Information - Mechanism-Based Small Molecule Probes for Labeling CD38 on Live Cells", JACS, 11 février 2009 (2009-02-11), pages 1-57, XP002760365, DOI: http://pubs.acs.org/doi/suppl/10.1021/ja80 8387g
- RUEGG UT: "Pharmacological prospects in the treatment of Duchenne muscular dystrophy", CURRENT OPINION IN NEUROLOGY, RAPID SCIENCE PUBLISHERS, LONDON, GB, vol. 26, no. 5, 1 octobre 2013 (2013-10-01), pages 577-584, XP009191136, ISSN: 1350-7540
- LOPEZ JOSE R ET AL: "Altered Ca2+ Homeostasis in Human Uremic Skeletal Muscle: Possible Involvement of cADPR in Elevation of Intracellular Resting [Ca2+]", NEPHRON, S. KARGER AG, SWITZERLAND, vol. 100, no. 4, 1 janvier 2005 (2005-01-01), pages p51-p60, XP009191127, ISSN: 0028-2766
- MORANDI ET AL.: "Dystrophin analysis in duchenne and becker muscular dystrophy carriers: Correlation with intracellular calcium and albumin", ANN NEUROL, 1990, pages 674-675, DOI: 10.1002/ana.410280512
- BOURKE ET AL: "Interventions for preventing and treating cardiac complications in Duchenne and Becker muscular dystrophy and X-linked dilated cardiomyopathy", COCHRANE DATABASE SYST REV., 2018, pages 1-3, DOI: 10.1002/14651858.CD009068.pub3.
- VOISIN ET AL: "Therapeutic strategies for Duchenne and Becker dystrophies", INT REV CYTOL., 2004, pages 1-30, DOI: 10.1016/S0074-7696(04)40001-1
- LUCAS-HÉRON ET AL: "Is there a maturation defect related to calcium in muscle mitochondria from dystrophic mice and Duchenne and Becker muscular dystrophy patients?", J NEUROL SCI., 1989, pages 299-306, DOI: 10.1016/0022-510x(89)90116-0
- GISSEL H ET AL: "The Role of Ca2+ in Muscle Cell Damage", ANN N Y ACAD SCI., 2005, pages 166-180, DOI: 10.1196/annals.1363.013
- COSKER ET AL: "The ecto-enzyme CD38 is a nicotinic acid adenine dinucleotide phosphate (NAADP) synthase that couples receptor activation to Ca2+ mobilization from lysosomes in pancreatic acinar cells", HE JOURNAL OF BIOLOGICAL CHEMISTRY, 2010, pages 38251-38259, DOI: 10.1074/jbc.M110.125864
- GRAEFF ET AL: "Acidic residues at the active sites of CD38 and ADP-ribosyl cyclase determine nicotinic acid adenine dinucleotide phosphate (NAADP) synthesis and hydrolysis activities", J BIOLOGICAL CHEMISTRY, 2006, pages 28951-28957, DOI: 10.1074/jbc.M604370200
- GRAEFF ET AL: "A novel cycling assay for nicotinic acid-adenine dinucleotide phosphate with nanomolar sensitivity", BIOCHEM. J., 2002, pages 163-168, DOI: 10.1042/BJ20020644
- CANCELA ET AL: "Coordination of agonist-induced Ca2+-signalling patterns by NAADP in pancreatic acinar cells", NATURE, 1999, pages 74-76, DOI: 10.1038/18032
- CHOE ET AL P: "CD38 exacerbates focal cytokine production, postischemic inflammation and brain injury after focal cerebral ischemia", PLOS ONE, 2011, pages 1-8, DOI: 10.1371/journal.pone.0019046
- RAE ET AL: "Cognitive dysfunction in Duchenne muscular dystrophy: a possible role for neuromodulatory immune molecules", J. NEUROPHYSIOL, 2016, pages 1304-1305, DOI: 10.1152/jn.00248.2016

## Description

La présente invention concerne le domaine de la médecine. Elle concerne plus particulièrement l'utilisation de composés pour prévenir et/ou traiter, chez un sujet, une myopathie, typiquement une dystrophie musculaire ou une cardiomyopathie, ou un traumatisme musculaire. L'invention concerne également les compositions, en particulier les compositions pharmaceutiques comprenant de tels composés, et kits correspondants, ainsi que leurs utilisations pour prévenir et/ou traiter une myopathie ou un traumatisme musculaire. Les composés et compositions selon l'invention peuvent typiquement être avantageusement utilisés pour prévenir et/ou traiter une dystrophie musculaire, une cardiomyopathie ou un traumatisme musculaire, de préférence la Dystrophie Musculaire de Duchenne (DMD), la Dystrophie Musculaire de Becker (DMB) et/ou un symptôme ou une anomalie caractéristique de la DMD ou de la DMB.

### ART ANTERIEUR

Les dystrophies musculaires sont un groupe de maladies qui se caractérisent par une faiblesse et une dégénérescence progressive des muscles du corps ainsi que par une inflammation. Ceux-ci s'atrophient peu à peu, c'est-à-dire qu'ils perdent leur force et leur volume. Ce sont des maladies génétiques qui peuvent commencer dès la naissance, pendant l'enfance ou encore à l'âge adulte. Une quarantaine de gènes différents sont impliqués dans les dystrophies musculaires. Il est difficile de connaître la fréquence de l'ensemble des dystrophies, mais certaines études estiment qu'environ 1 personne sur 3 500 est atteinte de DMD. Les dystrophies peuvent toucher tout le monde et apparaître à n'importe quel âge. Il en existe plus de 30 formes, qui diffèrent par l'âge d'apparition des symptômes, les muscles touchés et la gravité. Ce sont des maladies qui s'aggravent progressivement et pour lesquelles il n'existe pas de traitement. Les muscles touchés en cas de dystrophie musculaire sont principalement ceux qui permettent les mouvements volontaires, en particulier les muscles des jambes et des bras. Les muscles respiratoires et le coeur peuvent parfois être atteints. C'est le cas dans la DMD. Malheureusement, la plupart des personnes atteintes de dystrophie musculaire perdent peu à peu la capacité de marcher. D'autres symptômes peuvent s'associer à la faiblesse musculaire, notamment des troubles cardiaques, gastro-intestinaux, oculaires, et inflammatoires.

On distingue généralement deux grands groupes de dystrophies musculaires :
- les dystrophies musculaires congénitales (DMC), qui débutent dans les 6 premiers mois de la vie. Il en existe une dizaine de formes, de gravité variable, dont la DMC avec déficit primaire en mérosine (un constituant de la membrane des cellules du muscle), la DMC d'Ullrich, le syndrome de la colonne raide et le syndrome de Walker-Warburg ; et
- les dystrophies musculaires débutant plus tard dans l'enfance ou à l'âge adulte telles que notamment la dystrophie musculaire de Duchenne (DMD) ; la dystrophie musculaire de Becker (DMB) ; la dystrophie musculaire d'Emery-Dreyfuss (il en existe plusieurs formes) ; la dystrophie musculaire fascio-scapulo-humérale, aussi appelée myopathie de Landouzy-Déjerine ; les myopathies des ceintures, ainsi nommées, car elles touchent surtout les muscles situés autour des épaules et des hanches ; les dystrophies myotoniques (types I et II), dont fait partie la dystrophie de Steinert qui se caractérisent par une myotonie (i.e. les muscles ne parviennent pas à se relâcher normalement après une contraction) ; et la dystrophie musculaire oculo-pharyngée.

La plus connue et la plus fréquente des dystrophies musculaires est la dystrophie musculaire de Duchenne (DMD) aussi appelée myopathie de Duchenne. Elle a pour origine la perte de fonction d'un gène porté par le chromosome X dans la région p21 codant pour la dystrophine, une protéine située sous la membrane et jouant un rôle dans la contraction du muscle. La DMD est une maladie assez répandue dans le monde, n'affectant que le sexe masculin. Son incidence est de un sur 3500. A la naissance, aucun symptôme n'est observé, mais au cours du développement une perte graduelle de la motricité s'installe. La maladie se manifeste en effet par une dégénérescence progressive des muscles squelettiques, lisses et cardiaque ainsi que par des déficits cognitifs et comportementaux. Cette dégénérescence est en partie liée à une augmentation excessive du calcium qui conduit à l'activation de protéases à l'origine de la mort cellulaire. A l'âge de 12 ans, ces enfants perdent la marche et plus de la moitié ne vivent pas au-delà de 20-30 ans. Les causes de la mort sont soit une insuffisance respiratoire d'origine musculaire soit une cardiomyopathie. Il n'existe pas aujourd'hui de traitement efficace contre la DMD.

Les symptômes de la dystrophie musculaire de Becker sont comparables à ceux de la dystrophie de Duchenne, mais ils sont moins marqués et l'évolution est moins rapide. Les symptômes débutent vers 5 à 15 ans, parfois plus tard, et se caractérisent par une perte progressive de la force des muscles des membres et du tronc. Dans plus de la moitié des cas, la marche reste possible jusqu'à l'âge de 40 ans environ. En revanche, une atteinte du coeur est fréquente et peut apparaître dès le début.

Actuellement les seuls traitements utilisés contre les myopathies, en particulier contre les dystrophies musculaires, visent à réduire l'inflammation et la progression de la maladie à l'aide de glucorticoïdes (prednisone et prednisolone). Ces traitements ont des effets bénéfiques modestes et provoquent de nombreux effets secondaires qui peuvent mener à des complications, en particulier dans les traitements à long terme. Les effets secondaires incluent la prise de poids qui peut compromettre la mobilité, une réduction de la densité minérale osseuse accompagnée d'un risque accru de fractures, le développement d'une cataracte, ainsi qu'une augmentation de la pression intraoculaire. Les β-bloquants, les inhibiteurs de phosphodiesterase et les inhibiteurs de l'enzyme de conversion de l'angiotensine sont spécifiquement utilisés pour améliorer la fonction cardiaque, en particulier pour réduire le développement de l'hypertrophie cardiaque ainsi que l'arythmie observées chez le patient. Cependant, ces traitements n'ont pas d'effet sur la dégénérescence musculaire cardiaque ou squelettique. De plus ils sont utilisés tardivement, à des fins curatives, pour réduire les symptômes apparus et ne peuvent être utilisés de manière préventive.

Goody et al Plos Biology vol. 10, 10, 2012, e1001409 décrit NAD⁺ pour son utilisation dans le traitement de la myopathie ou d'un traumatisme musculaire.

Les inventeurs décrivent à présent des molécules ou composés, et compositions comprenant de tels molécules ou composés, lesdits composés, actifs sur l'ensemble de la musculature (muscles squelettiques, cardiaques et lisses), étant capables non seulement de traiter une myopathie diagnostiquée, ou un traumatisme musculaire, mais également de prévenir la survenue d'une myopathie. Ces composés sont en particulier capables de réduire les atteintes nécrotiques observées au niveau du coeur, du diaphragme et des muscles squelettiques des sujets atteints de DMD et DMB et de prévenir de telles atteintes chez les sujets à risque de développer une myopathie, typiquement une dystrophie musculaire.

### RESUME DE L'INVENTION

L'invention concerne des molécules (également désignées dans le présent texte à l'aide du terme « composés ») destinées à la prévention ou au traitement des myopathies, typiquement des dystrophies musculaires et des cardiomyopathies, et des traumatismes musculaires. Dans le cadre de la présente invention, les termes « myopathie » ou « myopathies » ne désignent pas et ne couvrent pas la myopathie urémique.

Les molécules de l'invention sont de préférence destinées à la prévention ou au traitement de la dystrophie musculaire de Duchenne (DMD) ou de la dystrophie musculaire de Becker (DMB) ou d'un symptôme ou d'une anomalie caractéristique de la DMD ou de la DMB.

Dans un mode de réalisation préféré de l'invention, le composé selon l'invention i) réduit ou inhibe l'activité des canaux calciques et réduit les atteintes fonctionnelles des cellules musculaires squelettiques, lisses et/ou cardiaques, ii) diminue la nécrose musculaire éventuellement présente des cellules musculaires squelettiques, lisses et/ou cardiaques, et de préférence iii) réduit l'inflammation musculaire.

Un composé particulier utilisable pour prévenir ou traiter une myopathie chez un sujet est un composé antagoniste des ADP ribosyl cyclases (CD38 étant un exemple d'ADP ribosyl cyclase), un composé antagoniste de l'ADP ribose cyclique (« cADPR » ou « ADPRc ») et/ou un composé antagoniste de l'acide nicotinique adénine dinucléotide phosphate (« NAADP » ou « nicotinic acid adenine di-nucleotide phosphate »).

L'invention concerne un composé antagoniste des ADP ribosyl cyclases pour une utilisation pour prévenir ou traiter une myopathie ou pour traiter un traumatisme musculaire chez un sujet, la myopathie n'étant pas une myopathie urémique, caractérisé en ce que l'ADP ribosyl cyclase est CD38 et en ce que le composé antagoniste du CD38 est un composé de formule (II) : 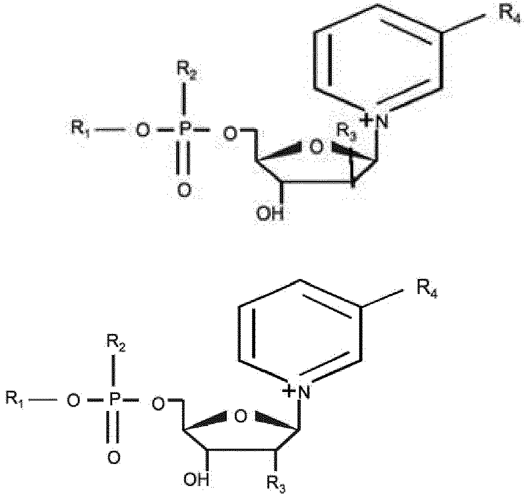 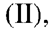 dans laquelle:
- R₁ = H, C₃H₇, C₄H₉, C₆H₁₃, C₁₂H₂₅ , groupe CH₂-benzene ou CH₂-CH₂-Benzene.
- R₂ = S, OH,
- R₃ = F, CN, Br, Cl et/ou
- R₄ = CONH₂, COOH, COCN, ou COF,

Ou le NAADP
et/ou mélange d'au moins deux desdits composés.

L'invention concerne également une composition pharmaceutique comprenant un composé selon l'invention et un support acceptable sur le plan pharmaceutique.

L'invention concerne par ailleurs un kit prophylactique ou thérapeutique comprenant composé selon l'invention, et/ou une composition selon la revendication 9, et au moins un composé sélectionné parmi un glucorticoïde tel que la prednizolone ou le deflazacort, un bétabloquant tel que l'albutérol, la diphénylhydantoïne (Dilantin^{®}), la méxilétine (Mexitil^{®}), le baclofène (Liorésal@), le dantrolène (Dantrium^{®}) ou la carbamazépine (Tégrétol^{®}), un inhibiteur de l'enzyme de conversion de l'angiotensine (ECA), et une séquence nucléotidique permettant de modifier l'expression de la dystrophine, lesdits composés se présentant de préférence dans des containers distincts.

Il est décrit un composé ou un mélange de composés tels que décrits dans le présent texte pour une utilisation pour traiter un traumatisme musculaire ou pour prévenir ou traiter une myopathie chez un sujet, typiquement une dystrophie musculaire et/ou une cardiomyopathie, ou au moins un symptôme ou au moins une anomalie caractéristique de celle(s)-ci, de préférence plusieurs symptômes ou anomalies caractéristiques de celle(s)-ci (par exemple 2, 3, 4 ou 5).

Il est par ailleurs décrit une composition, se présentant sous la forme d'une composition pharmaceutique, comprenant au moins un composé selon l'invention et un support acceptable sur le plan pharmaceutique. Un objet particulier concerne typiquement une composition pharmaceutique comprenant outre ledit au moins un composé selon l'invention, au moins un autre composé (différent des composés selon l'invention) actif sur le plan thérapeutique (et reconnu comme tel par l'homme de l'art).

Il est décrit également l'utilisation d'une telle composition pour prévenir ou traiter une myopathie ou pour traiter un traumatisme musculaire chez un sujet. Il est décrit aussi l'utilisation d'une telle composition pour traiter un traumatisme musculaire ou pour prévenir ou traiter une dystrophie musculaire ou une cardiomyopathie chez un sujet, typiquement au moins un symptôme ou une anomalie caractéristique de la dystrophie musculaire, de préférence plusieurs symptômes (par exemple 2, 3, 4 ou 5) ; de préférence pour prévenir ou traiter la DMD ou la DMB. Les utilisations décrites peuvent aussi être avantageusement mises en oeuvre en combinaison avec au moins un autre composé actif sur le plan thérapeutique (reconnu comme tel par l'homme de l'art et différent des composés selon l'invention) en particulier dans le traitement des dystrophies musculaires ou cardiomyopathies, typiquement d'au moins un symptôme ou d'une anomalie caractéristique de la DMD et/ou de la DMB.

Il est décrit enfin un kit prophylactique ou thérapeutique comprenant un composé (ou un mélange d'au moins deux, par exemple trois, composés selon l'invention), et/ou une composition selon l'invention, et au moins un autre composé actif sur le plan thérapeutique (reconnu comme tel par l'homme de l'art et différent des composés selon l'invention) en particulier dans le traitement des dystrophies musculaires, typiquement d'au moins un symptôme ou d'une anomalie caractéristique de la DMD et/ou de la DMB, d'une cardiomyopathie ou d'un traumatisme musculaire, lesdits composés se présentant de préférence dans des containers distincts.

### DESCRIPTION DETAILLEE

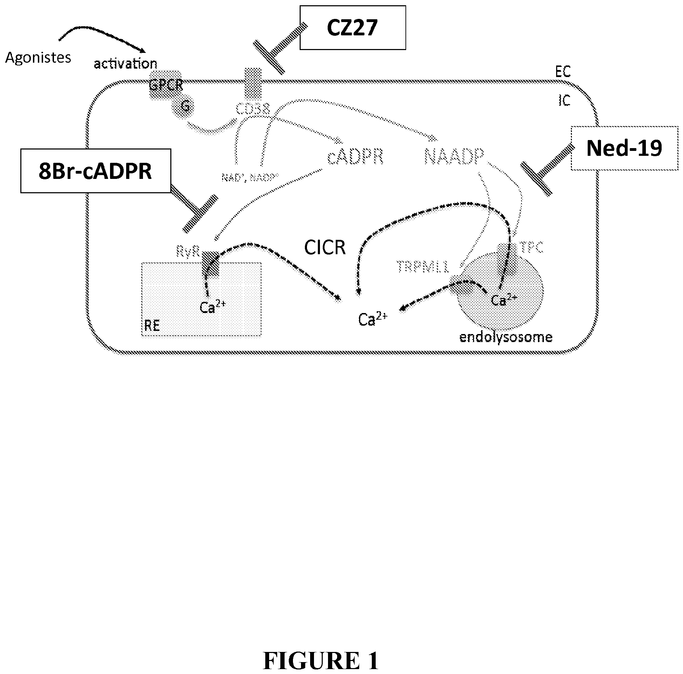

Les inventeurs ont démontré que CD38, et ses métabolites (cf. Figure 1) dont le NAADP, sont des cibles thérapeutiques clefs dans le traitement des myopathies et traumatismes musculaires, et que l'inhibition de la voie de CD38 permet en particulier de prévenir ou de traiter les atteintes nécrotiques du coeur et du diaphragme ainsi que l'atteinte fonctionnelle du muscle squelettique.

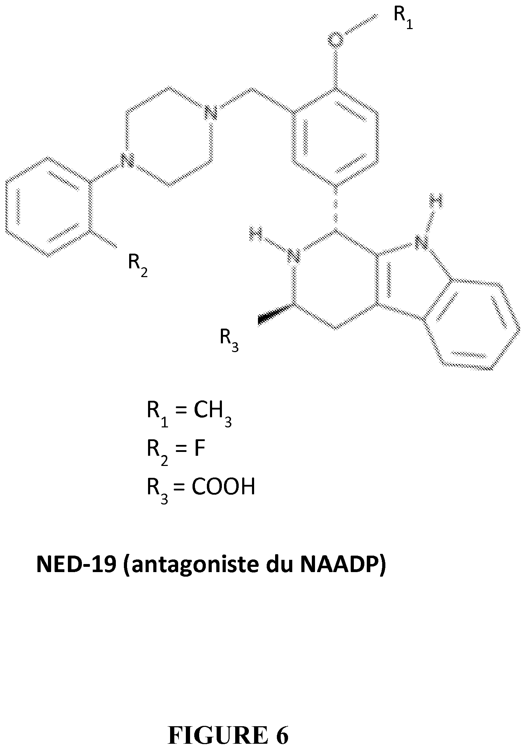

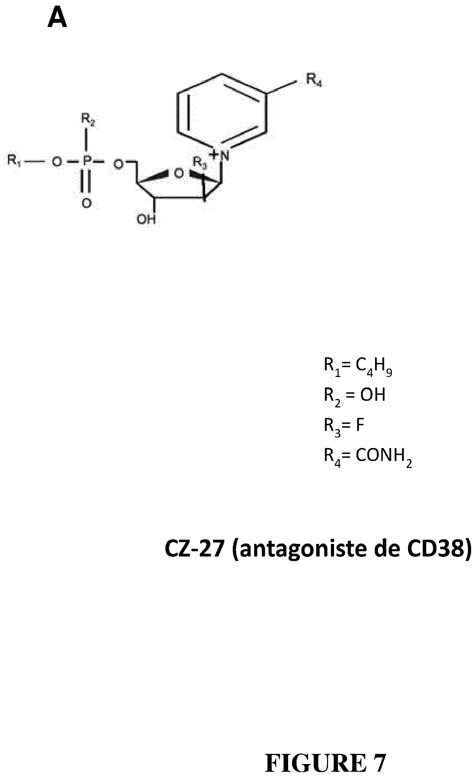

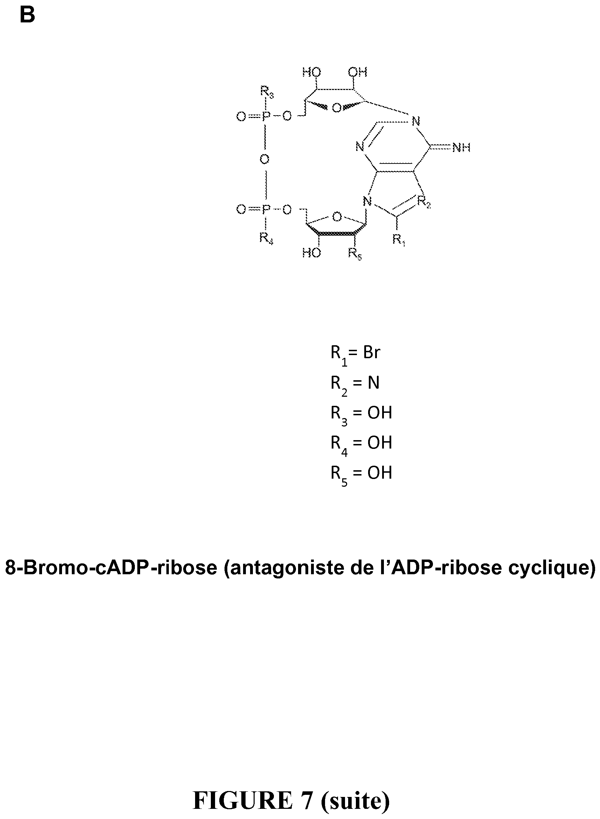

Pour apporter cette démonstration, les inventeurs ont développé un modèle de souris double mutant mdx/CD38^{-/-} (modèle de souris souffrant de myopathie de Duchenne et déficientes en CD38) puis ont réalisé une étude pharmacologique qui a permis d'identifier des composés capables de réduire ou inhiber l'activité des canaux calciques des cellules musculaires et de restaurer une fonctionnalité cellulaire comparable à celle rencontrée dans des conditions non pathologiques. L'efficacité des composés présélectionnés par les inventeurs a été testée et démontrée par ces mêmes inventeurs sur les cardiomyocytes d'un mammifère (i.e. sur les cardiomyocytes du modèle de souris) ainsi que sur une lignée de cellules de muscle (myotubes) humain provenant d'un patient souffrant de DMD. Les composés NED-19, 8-Bromo-cADPR, CZ-27 (cf. Figures 6, 7) et leurs dérivés fonctionnels ont ainsi été identifiés comme des composés directement utilisables dans la prévention et le traitement des myopathies en ce qu'ils sont en mesure de normaliser les paramètres clefs de l'homéostasie calcique du muscle, de réduire, de préférence supprimer, la nécrose et l'inflammation musculaire, et de restaurer la fonctionnalité du muscle squelettique, du muscle cardiaque et du diaphragme.

On décrit ainsi sur un composé antagoniste des ADP ribosyl cyclases (par exemple de CD38), un composé antagoniste de l'ADP ribose cyclique (cADPR) et/ou un composé antagoniste de l'acide nicotinique adénine dinucléotide phosphate (NAADP) (identifiés dans le présent texte en tant que « composés d'intérêt »), et/ou sur un mélange d'au moins deux desdits composés d'intérêt ou de leurs dérivés fonctionnels, par exemple trois, pour une utilisation pour prévenir ou traiter une myopathie, ou pour traiter un traumatisme musculaire, chez un sujet.

Des exemples de composés d'intérêt préférés i) réduisant ou inhibant l'activité des canaux calciques des cellules musculaires squelettiques, lisses et/ou cardiaques, ii) diminuant la nécrose musculaire éventuellement présente des cellules musculaires squelettiques, lisses et/ou cardiaques, et de préférence iii) réduisant l'inflammation musculaire, sont sélectionnés parmi le NED-19, le 8-bromo-cADPR, le CZ-27, un dérivé fonctionnel de ceux-ci et un mélange de ceux-ci, de préférence parmi le NED-19, le 8-bromo-cADPR, le CZ-27 et un mélange de ceux-ci, par exemple le mélange du NED-19 et du 8-bromo-cADPR ou le mélange du NED-19, du 8-bromo-cADPR et du CZ-27. Les dérivés fonctionnels desdits composés, en particulier les dérivés fonctionnels du NED-19, du 8-Bromo-cADPR, et du CZ-27 sont également capables d'exercer les fonctions i), ii) et de préférence iii) décrites ci-dessus et peuvent être considérés comme des exemples préférés supplémentaires.

Il est décrit par ailleurs un composé ou mélange de composés tels que décrits dans le présent texte pour une utilisation pour traiter un traumatisme musculaire, ou pour prévenir ou traiter une myopathie chez un sujet, typiquement une dystrophie musculaire et/ou une cardiomyopathie, ou au moins un symptôme ou au moins une anomalie caractéristique de celles-ci, de préférence plusieurs symptômes ou anomalies caractéristiques de celles-ci (par exemple 2, 3, 4 ou 5).

Un composé particulier utilisable dans une méthode de prévention ou de traitement telle que décrite dans le présent texte est un composé antagoniste des ADP ribosyl cyclases, typiquement de CD38.

Un composé antagoniste du CD38 préféré est avantageusement choisi parmi un composé un composé de formule (II) : 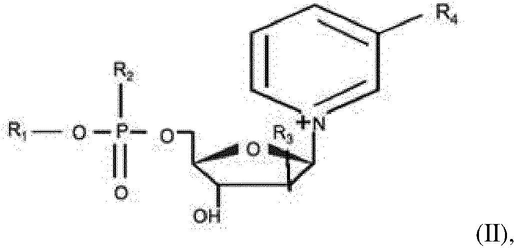 dans lequel :
- R₁ = H, C₃H₇, C₄H₉, C₆H₁₃, C₁₂H₂₅, groupe CH₂-benzene ou CH₂-CH₂-Benzene.
- R₂ =S, OH,
- R₃ = F, CN, Br, Cl et/ou
- R₄ = CONH₂, COOH, COCN, ou COF.

Le nicotinamide est avantageusement utilisé en tant qu'antagoniste du CD38 chez le sujet à traiter à une concentration d'au moins 10 mmolaires pour les applications décrites dans le cadre de la présente description.

Dans un mode de réalisation particulier, l'antagoniste du CD38 est choisi parmi le CZ-17 dans lequel R₁ = H, R₂ = O, R₃ = F et R₄ = CONH₂ ; le CZ-27 [2'-Deoxy-2'-fluoro-β-nicotinamide arabinofuranoside-5'-(n-butyl) phosphate)] dans lequel R₁ = C₄H₉, R₂ = OH, R₃ = F et R₄ = CONH₂ ; le CZ-57 dans lequel R₁ = C₃H₇, R₂ = OH, R₃ = F et R₄ = CONH₂ ; le CZ-45 dans lequel R₁ = C₆H₁₃, R₂ = OH, R₃ = F et R₄ = CONH₂ ; le CZ-48 dans lequel R₁ = H, R₂ = S, R₃ = F et R₄ = CONH₂ et le CZ-53 dans lequel R₁ = C₁₂H₂₅, R₂ = OH, R₃ = F et R₄ = CONH₂.

Dans un mode de réalisation particulier préféré, l'antagoniste du CD38 est choisi parmi le CZ-27, le CZ-48,. Un antagoniste particulièrement préféré du CD38 est le CZ-27.

Un autre composé particulier est un composé antagoniste du cADPR, de préférence un composé de formule (III) : 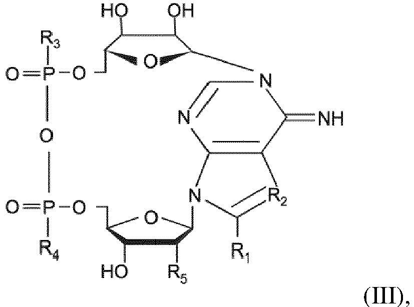 dans laquelle :
- R₁ = H, Br ou NH₂,
- R₂ = N ou C,
- R₃ = OH, F, CN ou un groupement CH₃COOCH₂O,
- R₄ = OH, F, CN ou un groupement CH₃COOCH₂O, et/ou
- R₅ = OH, F ou H₂PO₃,
ou un dérivé fonctionnel de celui-ci.

Plus particulièrement, l'antagoniste du cADPR est le 8-Bromo-cADPR (C₁₅H₂₀BrN₅O₁₃P₂ ou 8 Bromo adénosine diphosphate ribose cyclique) dans lequel R₁ = Br, R₂ = N, R₃ = OH, R₄ = OH et R₅ =OH) ou un dérivé fonctionnel de celui-ci. Le 7-deaza-8-Bromo-cADPR [dans lequel R₁ = Br, R₂ = C, R₃ = OH, R₄ = OH, et R₅ =OH] et le 8 amino-adénosine diphosphate ribose cyclique [dans lequel R₁ = NH₂, R₂ = N, R₃ = OH, R₄ = OH, et R₅ =OH] sont des exemples de dérivés fonctionnel du 8-Bromo-cADPR.

Un autre composé particulier est un composé antagoniste du NAADP, par exemple le NAADP lui-même : 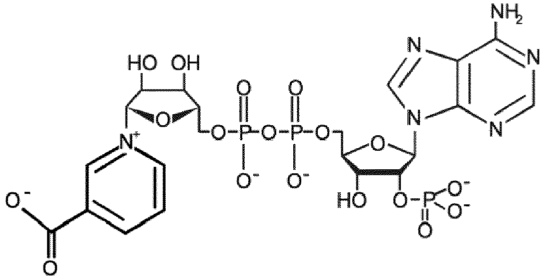 utilisé chez le sujet à traiter à une concentration d'au moins 100 µmolaires pour les applications décrites dans le cadre de la présente invention. Un autre composé antagoniste du NAADP est un composé de formule (IV) : 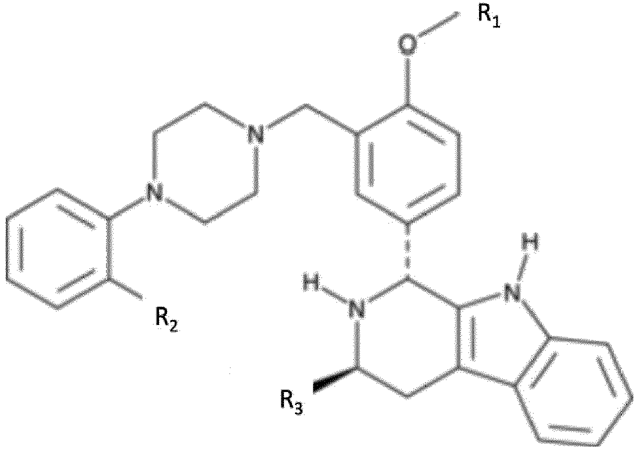 dans laquelle :
- R₁ = CH3 ou CN
- R₂ = F, CN, Br, et/ou
- R₃ = COOR₄ où R₄= H ou CH₃,
ou un dérivé fonctionnel dudit composé de formule (IV).

Plus particulièrement, l'antagoniste du NAADP est le NED-19 [C₃₀H₃₁FN₄O₃ ou trans-NED 19 ou (1R,3S)-1-[3-[[4-(2-Fluorophenyl)piperazin-1-yl]methyl]-4-methoxyphenyl]-2,3,4,9-tetrahydro-1H-pyrido[3,4-b]indole-3-carboxylic acid] dans lequel R₁ = CH₃, R₂ = F et R₃ = COOH, ou un dérivé fonctionnel de celui-ci.

De manière encore plus particulère l'antagoniste du NAADP est choisi parmi le NED-K dans lequel R₁ = CH₃, R₂ = CN et R₃ = COOH, le NED-19.4 dans lequel R₁ = CH₃, R₂ = F et R₃ = COOCH₃, et un dérivé fonctionnel de ceux-ci.

Le NED-K et le NED-19.4 sont des exemples de dérivés fonctionnels de NED-19.

Par « dérivé fonctionnel » on entend, , un composé capable i) de réduire ou inhiber l'activité des canaux calciques et de réduire les atteintes fonctionnelles des cellules musculaires squelettiques, lisses et/ou cardiaques, ii) de diminuer la nécrose musculaire éventuellement présente des cellules musculaires squelettiques, lisses et/ou cardiaques, et de préférence iii) de réduire l'inflammation musculaire.

Le dérivé fonctionnel est de préférence capable d'améliorer la structure des muscles squelettiques, du muscle cardiaque et du diaphragme, ainsi que la fonction de muscles squelettiques d'une « souris mdx » comme décrit dans la partie expérimentale de la présente demande de brevet. L'amélioration de la structure des muscles et du diaphragme sera validée par une diminution de la nécrose cellulaire mise en évidence par une coloration histologique (cf. Vianello et al., 2014, Neurobiol Dis.; 71:325-333). par exemple du collagène, dans les tissus des « souris mdx » traitées. L'amélioration de la fonction des muscles squelettiques pourra être mesurée à l'aide d'un newton mètre pour déterminer la force d'agrippement de la « souris mdx » traitée (cf. Vianello et al., 2014, FASEB J.; 28(6):2603-2619). L'amélioration de la résistance à la fatigue des muscles squelettiques pourra être mesurée par le test de la grille (chronométrage du temps de maintien d'une souris sur une grille renversée, Vianello et al., 2014, FASEB J.; 28(6):2603-2619).

Etant bien établi que la nécrose musculaire est due à une augmentation excessive du calcium musculaire, l'efficacité du dérivé fonctionnel devra passer par une amélioration des paramètres de l'homéostasie calcique, analysés par exemple par microscopie confocale (cf. Sarma et al., 2010, Proc. Natl. Acad. Sci. U.S.A. 107, 13165-13170 ; Vianello et al., 2014, FASEB J.; 28(6):2603-2619), dans des cardiomyocytes de souris et des myotubes de patient DMD:
- dans le cardiomyocyte de « souris mdx », il a été montré que les réserves calciques du réticulum sarcoplasmique étaient réduites, probablement en raison d'une fuite du Ca²⁺ via les RyRs qui sont anormalement sensibilisés. De plus, une augmentation de l'activité calcique spontanée de type spark et/ou vague a été observée dans ces cellules. A titre d'exemple le dérivé fonctionnel doit réguler l'activité spontanée calcique des cardiomyocytes de « souris mdx » en inversant au moins un, de préférence plusieurs paramètres délétères chez la « souris mdx » (sparks et vagues calciques spontanés, sensibilisation des RyRs, etc.). Le dérivé fonctionnel doit permettre de réduire le nombre de cardiomyocytes présentant de l'activité calcique spontanée ainsi que la fréquence des vagues calciques spontanées. Idéalement, aucune activité calcique spontanée des cardiomyocytes ne doit être observée. Si une telle activité peut néanmoins être mesurée, alors la fréquence des vagues calciques spontanées doit être significativement réduite.
- les myotubes en culture, préparés à partir de muscle squelettique de patients DMD humains, présentent une importante activité calcique spontanée sous forme de vagues calciques. A titre d'exemple, après préincubation avec le dérivé fonctionnel, l'amplitude des vagues calciques doit être significativement réduite. De plus, le nombre de myotubes présentant une activité calcique spontanée sera réduit d'une manière dose-dépendante pour des concentrations autour de 1 à 300 µM.

Un objet particulier concerne un composé selon l'invention pour une utilisation pour prévenir ou traiter la DMD ou la DMB.

Un composé particulièrement préféré est le NED-19 dont les inventeurs ont démontré, qu'il est particulièrement efficace pour prévenir l'activité calcique spontanée dans les cardiomyocytes et les myotubes humains de patient souffrant de dystrophie musculaire, en particulier de DMD.

Un autre composé selon l'invention particulièrement préféré est le CZ-27, dont les inventeurs ont démontré l'efficacité pour réduire l'amplitude des vagues calciques spontanées dans les myotubes humains de patient DMD.

Encore un autre composé particulièrement préféré est le 8 Bromo-cADPR , dont les inventeurs ont démontré l'efficacité pour réduire l'amplitude des vagues calciques spontanées dans les myotubes humains de patient DMD.

Dans un mode de réalisation préféré, le NED-19 et le 8 Bromo-cADPR, ou une combinaison de dérivés fonctionnels de ceux-ci, sont utilisés pour prévenir ou traiter une myopathie ou pour traiter un traumatisme musculaire chez un sujet.

Les molécules identifiées par les inventeurs, et leurs dérivés fonctionnels, ont notamment en commun de réduire l'activité calcique délétère et l'inflammation observées dans les myopathies, en particulier dans les dystrophies musculaires telles que la DMD et la DMB. Comme expliqué dans la partie expérimentale, les inventeurs ont en effet démontré notamment l'obtention, à l'aide desdites molécules, d'une amélioration des paramètres de l'homéostasie calcique de la DMD.

Un avantage considérable que présentent les molécules (ou composés) de l'invention sélectionnées par les inventeurs, par rapport aux molécules utilisées dans l'art antérieur, est que contrairement à ces dernières, elles sont en mesure de lutter très significativement contre la nécrose musculaire quelle que soit la nature du/des muscles concernés (squelettiques, cardiaques et/ou lisses) et peuvent être utilisées de manière préventive en amont de tout symptôme, en particulier chez les sujets présentant une prédisposition au développement d'une myopathie. Elles ne sont en outre associées à aucun des effets secondaires répertoriés pour les traitements existants, tels que la prise de poids et les fractures.

Le sujet concerné est un animal, typiquement un mammifère, par exemple un mammifère choisi parmi un être humain, une souris, un rat, un porc et un chien. Le sujet concerné est de préférence un être humain quel que soit son genre. L'être humain concerné est typiquement un être humain de sexe mâle dans le cas des myopathies liées à l'X. La mutation étant récessive, un homme porteur de l'allèle mutant sera malade car hémizygote pour le chromosome X. La présence des deux allèles mutants est en revanche nécessaire pour que la maladie s'exprime chez une fille.

Dans le contexte de la présente description, les myopathies dont la prévention ou le traitement est recherchée sont des atteintes directes d'un des constituants du tissu musculaire. Elles se traduisent par une dégénérescence dudit tissu musculaire. Des exemples typiques de myopathies sont les dystrophies musculaires et les cardiomyopathies. Certaines myopathies sont d'origine génétique (liée à l'X, à transmission dominante ou à transmission récessive) d'autres sont acquises.

Les myopathies liées à l'X comprennent par exemple la dystrophie musculaire de Duchenne (DMD), la dystrophie musculaire de Becker (DMB), la dystrophie musculaire d'Emery-Dreyfus, la myopathie vacuolaire avec glycogénose autophagique (ou myopathie avec authophagie excessive).

Les myopathies à transmission dominante comprennent les dystrophies musculaires des ceintures de type 1A, 1B, 1C, 1D et 1E, la dystrophie myotonique de Steinert, la dystrophie musculaire fascio-scapulo-humérale de type 1A et 1B, la dystrophie musculaire oculopharyngée, la myopathie oculopharyngo distale, la dystrophie musculaire d'Emery-Dreyfus autosomique dominante, la myopathie de Bethléem, la myopathie avec surcharge en desmine, la dystrophie musculaire tibiale (ou myopathie distale de Udd), la myopathie distale de Welander, la myopathie distale de Laing, la myopathie distale avec faiblesse des cordes vocales et du pharynx, la fibrose congénitale des muscles oculo-moteurs extrinsèques, la myopathie à corps d'inclusion et l'hypertrophie musculaire en rapport avec un trouble de la myostatine.

Les myopathies à transmission récessive comprennent les dystrophies musculaires des ceintures de type 2A, 2B, 2C, 2D, 2E, 2F, 2G, 2H et 2I, la myopathie distale de type Nonaka (ou myopathie distale à vacuoles bordées), la myopathie distale de type Miyoshi, la dystrophie musculaire oculopharyngée autosomique récessive, la myopathie à corps d'inclusion 2, la dystrophie musculaire autosomique récessive associée à une epidermose bulleuse, la dystrophie musculaire congénitale par déficit en mérosine, la dystrophie musculaire congénitale par déficit en intégrine, la dystrophie musculaire congénitale avec raideur de la colonne vertébrale, le syndrome Muscle-Eye-Brain, le syndrome de Walker-Warburg et la dystrophie musculaire d'Emery-Dreyfuss de type 3.

Les myopathies congénitales avec anomalies structurelles et les myopathies inflammatoires acquises sont également comprises dans la définition du terme « myopathies » tel qu'utilisé dans le présent texte.

Un composé ou mélange de composés selon l'invention est avantageusement utilisé pour prévenir ou traiter au moins un symptôme de la dystrophie musculaire, de préférence au moins deux ou trois symptômes, lesdits symptômes étant sélectionnés parmi une dégénérescence musculaire, une faiblesse musculaire (hypotonicité), une myotonie, une insuffisance cardiaque d'origine musculaire, une insuffisance respiratoire d'origine musculaire, une inflammation et un retard mental ; pour prévenir ou traiter au moins un symptôme d'une cardiomyopathie, de préférence au moins deux ou trois symptômes, lesdits symptômes étant sélectionnés parmi arythmie, tachycardie, asthénie et dyspnée ; ou pour prévenir ou traiter au moins un symptôme du traumatisme musculaire, de préférence au moins deux ou trois symptômes, lesdits symptômes étant sélectionnés parmi une contusion, une courbature et une contracture.

Des exemples de composés d'intérêt préférés, pour prévenir ou traiter une myopathie, en particulier la DMD ou la DMB, ou un traumatisme musculaire, sont identifiés ci-dessous :
- le NED-19 [C₃₀H₃₁FN₄O₃ ou trans-NED 19 ou (1R,3S)-1-[3-[[4-(2-Fluorophenyl)piperazin-1-yl]methyl]-4-methoxyphenyl]-2,3,4,9-tetrahydro-1H-pyrido[3,4-b]indole-3-carboxylic acid] présentant la formule ci-dessous : 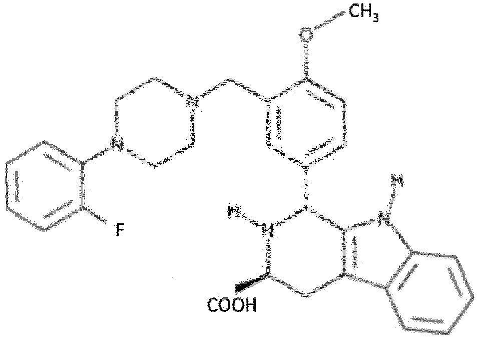
- le 8-Bromo-cADPR (C₁₅H₂₀BrN₅O₁₃P₂ ou 8 Bromo adénosine diphosphate ribose cyclique), présentant la formule ci-dessous : 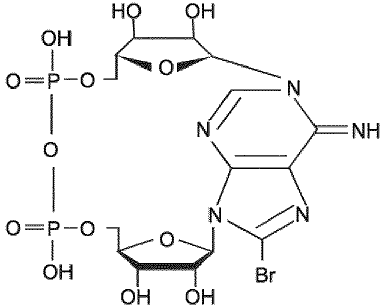
- le CZ-27 [2'-Deoxy-2'-fluoro-β-nicotinamide arabinofuranoside-5'-(n-butyl) phosphate], présentant la formule ci-dessous :

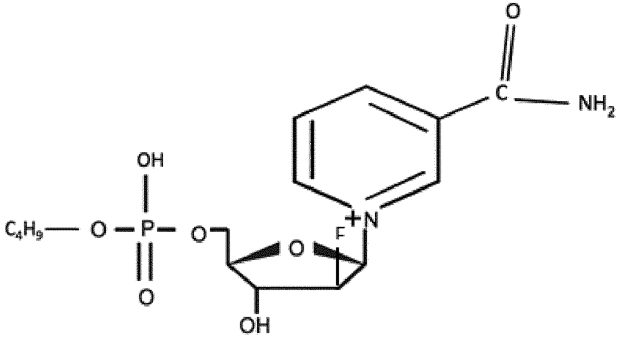

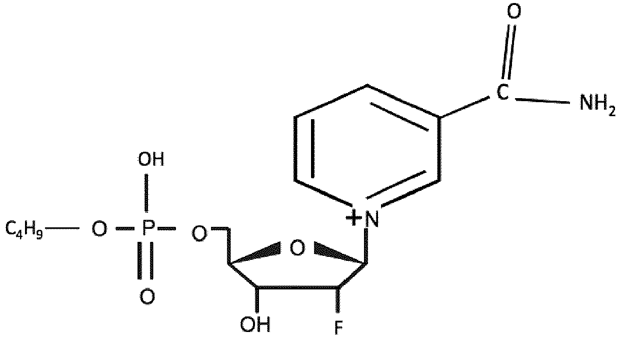

Des dérivés fonctionnels préférés de composés d'intérêt pour prévenir ou traiter une myopathie ou un traumatisme musculaire sont choisis par exemple parmi le NED-K, le NED-19.4, le CZ-17, le CZ-45, le CZ-48, le CZ-53, le CZ-57, le 7-deaza-8-Bromo-cADPR et le 8 amino-adénosine diphosphate ribose cyclique, de préférence parmi le NED-K, le NED-19.4, le 7-deaza-8-Bromo-cADPR et le 8 amino-adénosine diphosphate ribose cyclique. Ces dérivés présentent les mêmes propriétés fonctionnelles, listées plus haut dans le texte, que les composés d'intérêt dont ils dérivent.

Il est également décrit une méthode de criblage de molécules ou composés d'intérêt pour prévenir ou traiter une myopathie ou pour traiter un traumatisme musculaire. Cette méthode comprend une étape d'évaluation de la capacité de composés tests à i) réduire ou inhiber l'activité des canaux calciques des cellules musculaires squelettiques, lisses et/ou cardiaques, ii) diminuer la nécrose musculaire des cellules musculaires squelettiques, lisses et/ou cardiaques, et de préférence iii) à réduire l'inflammation musculaire. La méthode peut être mise en oeuvre en présence de l'un, ou de plusieurs, des composés d'intérêt selon l'invention afin de tester la capacité desdits composés tests à amplifier l'action desdits composés d'intérêt ou à agir en synergie avec eux. De telles méthodes peuvent être mises en oeuvre de manière automatisée. Des exemples de telles méthodes sont décrites dans le brevet US 5,429,921. Les composés pharmacologiques tests peuvent provenir de banques de composés chimiques ou naturels. Typiquement, plusieurs essais impliquant différentes concentrations de composés tests peuvent être réalisés en parallèle afin d'évaluer les réponses obtenues aux différentes concentrations, l'une desdites concentrations, typiquement l'absence de composé test (concentration zéro) ou une concentration de composé test inférieure à la limite de détection, servant de témoin négatif. Les réactifs adaptés à la mise en oeuvre de telles méthodes sont connus de l'homme du métier.

Dans un mode de réalisation préféré de l'invention, les composés d'intérêt sont utilisés pour prévenir et/ou traiter une pathologie choisie parmi un traumatisme musculaire, une myopathie, en particulier une dystrophie musculaire ou une cardiomyopathie, et/ou un symptôme ou une anomalie caractéristique d'une telle myopathie, de préférence pour prévenir ou traiter la DMD ou la DMB.

Dans un mode de réalisation particulier de l'invention, les composés d'intérêt sont utilisés pour prévenir et/ou traiter au moins un symptôme des myopathies, de préférence au moins deux ou trois symptômes, lesdits symptômes étant sélectionnés parmi une dégénérescence musculaire, une faiblesse musculaire (hypotonicité), une myotonie, une insuffisance cardiaque d'origine musculaire, une insuffisance respiratoire d'origine musculaire, une inflammation et un retard mental de préférence parmi une dégénérescence musculaire, une myotonie, une insuffisance cardiaque d'origine musculaire, une insuffisance respiratoire d'origine musculaire et une inflammation.

Comme expliqué précédemment, les approches thérapeutiques actuelles des myopathies faisant intervenir des corticoïdes ne retardent la marche que de 3 ans, entrainent une prise de poids qui peut compromettre la mobilité, une réduction de la densité minérale osseuse avec le risque accru de fractures, la survenue de cataracte, ainsi qu'une augmentation de la pression intraoculaire. Les β-bloquants, les inhibiteurs de phosphodiesterase et les inhibiteurs de l'enzyme de conversion de l'angiotensine n'ont quant à eux pas d'effet sur la dégénérescence musculaire cardiaque ou squelettique. De plus, ils sont généralement utilisés tardivement, à des fins curatives, pour réduire les symptômes apparus et ne peuvent être utilisés de manière préventive. Il n'existe en particulier à ce jour aucune molécule ou composé permettant de traiter, idéalement de prévenir, de manière globale, les nécroses affectant les muscles squelettiques, lisses et cardiaques.

Dans un mode de réalisation préféré de l'invention, au moins un composé d'intérêt tel que décrit dans le présent texte est utilisé pour prévenir et/ou traiter une dystrophie musculaire, telle que la DMD ou la DMB, ou pour prévenir et/ou traiter une cardiomyopathie, ou pour traiter un traumatisme musculaire. Le NED-19, 8-Bromo-cADPR et le CZ-27 sont des exemples de composés d'intérêt utilisés de manière préférée pour prévenir et/ou traiter ces pathologies. A noter que les dérivés fonctionnels de ces composés sont eux-mêmes des composés préférés au sens de l'invention. Des mélanges de composés d'intérêt tels que décrits dans le présent texte, ou de leurs dérivés fonctionnels, peuvent également être utilisés, tels qu'un mélange Ned-19/8-Bromo-cADPR, NED-19/CZ-27, 8-Bromo-cADPR/CZ-27 ou NED-19/8-Bromo-cADPR/CZ-27.

Ce au moins un composé d'intérêt, ou ce mélange de composés d'intérêt, peut être utilisé, dans un mode de réalisation particulier de l'invention, en combinaison avec un composé distinct connu de l'homme du métier et utilisé classiquement :
- dans le traitement des dystrophies musculaires, ledit composé distinct étant de préférence sélectionné parmi un glucorticoïde tel que la prednisolone et le deflazacort, un bétabloquant tel que l'albutérol, la diphénylhydantoïne (Dilantin^{®}), la méxilétine (Mexitil^{®}), le baclofène (Liorésal), le dantrolène (Dantrium^{®}), la carbamazépine (Tégrétol^{®}), et un inhibiteur de l'enzyme de conversion de l'angiotensine (ECA) ;
- dans le traitement des cardiomyopathies, ledit composé distinct étant de préférence sélectionné parmi l'acébutolol (Sectral^{®}), l'albutérol, la diphénylhydantoïne (Dilantin^{®}), la méxilétine (Mexitil^{®}), le baclofène (Liorésal^{®}), le dantrolène (Dantrium^{®}), la carbamazépine (Tégrétol^{®}); ou
- dans le traitement des traumatismes musculaires, ledit composé distinct étant de préférence sélectionné parmi un tonique veineux ou anti-oedémateux et un anti-inflammatoire.

Le veinotonique peut être sélectionné parmi un anthrocyanoside, un citroflavonoïde et un flavonoïde, par exemple parmi Adenyl^{®}, Ampecyclal^{®}, Arkogelules marronnier d'Inde^{®}, Bioveinal Elixir^{®}, Cyclo 3^{®}, Climaxol^{®}, Daflon^{®}, Difrarel^{®}, Diovenor^{®}, Doxium^{®}, Esbériven^{®}, Flavan^{®}, Flebosmil^{®}, Ginkor^{®}, Hirucrème^{®}, Intercyton^{®}, Madécassol^{®}, Mediveine^{®}, Phlebogel^{®}, Phytomélis^{®}, Relvène^{®}, Rhéoflux^{®}, Thlascose^{®}, Veinamitol^{®}, Veinobiase^{®}, Veliten^{®}, Vivène ^{®}, Tisane phlébosedol^{®}, Santane V3^{®}, et Fluon ^{®}.

Le médicament anti-inflammatoire est typiquement à base de diclofénac ou d'ibuprofène, ces traitements permettant d'observer des résultats satisfaisants dans la prise en charge de la douleur, de la rougeur et de la chaleur consécutives à la contusion. L'anti-inflammatoire peut être sélectionné par exemple parmi AdvilGel 5 %^{®}, Algésal suractivé^{®}, Arnica complexe N°1^{®}, Arnicagel^{®}, et Arnicalme^{®}.

Un autre objet de l'invention concerne par ailleurs une composition se présentant sous la forme d'une composition pharmaceutique comprenant au moins un composé d'intérêt selon l'invention, ou mélange de composés d'intérêt selon l'invention, et un support acceptable sur le plan pharmaceutique (identifiée dans le présent texte en tant que « composition d'intérêt »). Un objet particulier concerne typiquement une composition pharmaceutique comprenant outre ledit au moins un composé d'intérêt selon l'invention, ou ledit mélange de composés d'intérêt selon l'invention, au moins un autre composé (différent des composés d'intérêt utilisés dans le contexte de l'invention pour prévenir ou traiter une myopathie) actif sur le plan thérapeutique (et reconnu comme tel par l'homme de l'art), tel qu'un adjuvant immunologique ou un composé actif dans la prévention ou le traitement, typiquement le traitement, d'un symptôme ou d'une anomalie caractéristique d'une myopathie (tels que décrits dans le présent texte par exemple).

L'invention concerne également une composition telle que décrite dans le présent texte pour une utilisation pour traiter un traumatisme musculaire ou pour prévenir ou traiter une myopathie, typiquement une pathologie choisie parmi une dystrophie musculaire, ou un symptôme ou une anomalie caractéristique de ladite dystrophie musculaire, et une cardiomyopathie, de préférence pour prévenir ou traiter la DMD ou la DMB.

Le terme « traitement » désigne le traitement curatif, symptomatique ou préventif. Les composés de la présente invention peuvent ainsi être utilisés chez des sujets (comme les mammifères, en particulier humains) atteints d'une maladie déclarée. Les composés de la présente invention peuvent aussi être utilisés pour retarder ou ralentir la progression ou prévenir une progression plus en avant de la maladie, améliorant ainsi la condition des sujets. Les composés de la présente invention peuvent enfin être administrés en « prévention » aux sujets non malades, mais qui pourraient développer normalement la maladie ou qui présentent un risque important de développer la maladie tels que les sujets prédisposés, en particulier les sujets prédisposés génétiquement.

Le ou les composés d'intérêt ou compositions selon l'invention peuvent être administrés de différentes manières et sous différentes formes.

Ainsi, dans un mode de réalisation typique, le ou les composés d'intérêt sont administrés au sujet, ensemble ou séparément, et le ou les composés d'intérêt ou compositions selon l'invention sont administrés de manière continue ou séquentielle, une ou plusieurs fois par jour (administration quotidienne), une ou plusieurs fois par semaine (administration hebdomadaire), ou une ou plusieurs fois par mois (administration mensuelle), pendant toute la durée du traitement, *i.e.* jusqu'à l'amélioration des symptômes de la pathologie traitée, de préférence la disparition de tout ou partie desdits symptômes.

Si nécessaire, la dose journalière peut par exemple être administrée en deux, trois, quatre, cinq, six ou plus, prises par jour ou par sous-doses multiples administrées par intervalles appropriés pendant la journée.

Ces composés ou compositions peuvent être par exemple administrés de manière systémique, par voie orale, parentérale, par inhalation ou par injection, comme par exemple par voie intraveineuse, intra-péritonéale, intra-musculaire, sous-cutanée, trans-dermique, intra-artérielle, etc. S'agissant d'un traitement à long terme, la voie d'administration préférée sera sublinguale, orale, intra-musculaire, ou transcutanée.

Les compositions peuvent être formulées sous forme de suspensions injectables, huiles, suppositoires, gélules, capsules, aérosols, etc., éventuellement au moyen de formes galéniques ou de dispositifs assurant une libération prolongée et/ou retardée. Pour les injections, les composés sont généralement conditionnés sous forme de suspensions liquides, qui peuvent être injectées au moyen de seringues ou de perfusions, par exemple.

Il est entendu que le débit et/ou la dose injectée peuvent être adaptés par l'homme du métier en fonction du patient, de la pathologie, du mode d'administration, etc. En général, la dose journalière du composé sera la dose minimum pour obtenir l'effet thérapeutique.

La quantité de composé présente dans la composition thérapeutique peut être modulée de façon à obtenir un taux circulant de principe actif nécessaire à l'obtention de l'effet thérapeutique désiré pour un patient particulier, une composition, un mode d'administration, et ce de préférence sans toxicité pour le patient. La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.

Typiquement, les composés sont administrés au sujet à traiter à des doses pouvant varier entre 10 mg et 1 g par Kg, de préférence entre 20 mg et 0,5 g par Kg. D'autre part, les compositions selon l'invention peuvent comprendre, en outre, d'autres agents ou principes actifs comme expliqué précédemment. Les compositions selon l'invention peuvent aussi comprendre un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc.

On décrit aussi les méthodes de prévention ou de traitement d'une myopathie chez un sujet, typiquement d'une dystrophie musculaire ou d'une cardiomyopathie, comprenant l'administration à un sujet souffrant d'une myopathie ou susceptible de développer une myopathie d'un composé ou d'une composition d'intérêt tels que décrits dans le présent texte pour prévenir ou traiter ladite myopathie.

Elle concerne par ailleurs les méthodes de prévention ou de traitement d'un sujet atteint d'une myopathie telle que décrite dans le présent texte, typiquement d'une dystrophie musculaire ou d'une cardiomyopathie, ou d'un sujet atteint d'un traumatisme musculaire. Ces méthodes comprennent toutes une étape d'administration à un sujet souffrant d'une telle pathologie ou susceptible de développer une telle pathologie d'un composé ou d'une composition d'intérêt tels que décrits dans le présent texte pour prévenir ou traiter ladite pathologie.

L'invention concerne enfin un kit prophylactique ou thérapeutique comprenant un composé ou une composition selon l'invention et au moins un composé additionnel tel qu'un adjuvant immunologique ou un composé actif sur le plan prophylactique ou thérapeutique (reconnu comme tel par l'homme de l'art et différent des composés selon l'invention) en particulier un composé actif dans le traitement des traumatismes musculaires, cardiopathies, ou dystrophies musculaires, typiquement d'au moins un symptôme ou d'une anomalie caractéristique de la DMD et/ou de la DMB, lesdits composés se présentant de préférence dans des containers distincts, de préférence stériles.

Dans un kit particulier, le composé selon l'invention est un composé sélectionné parmi un antagoniste de l'ADP ribosyl cyclase, typiquement de CD38, un antagoniste du cADPR, et/ou un antagoniste du NAADP, et un mélange de ceux-ci, de préférence un composé sélectionné parmi NED-19, le 8-Bromo-cADPR, le CZ-27, un dérivé fonctionnel de l'un desdits composés et un mélange de ceux-ci.

Dans un kit particulier, le au moins un composé distinct actif sur le plan thérapeutique en particulier dans le traitement des dystrophies musculaires est sélectionné parmi un glucorticoïde tel que la prednizolone ou le deflazacort, un bétabloquant tel que l'albutérol, la diphénylhydantoïne (Dilantin^{®}), la méxilétine (Mexitil^{®}), le baclofène (Liorésal), le dantrolène (Dantrium^{®}) ou la carbamazépine (Tégrétol^{®}), un inhibiteur de l'enzyme de conversion de l'angiotensine (ECA) et une séquence nucléotidique permettant de modifier l'expression de la dystrophine, typiquement de restaurer l'expression physiologique (vs pathologique) de la dystrophine [une telle séquence peut se présenter sous la forme d'un oligonucléotide anti-sens, d'un morpholino, d'un oligomère tricyclo-ADN (« tcDNA »), d'un vecteur, par exemple d'un vecteur AAV porteur d'un transgène U7, etc.].

Le kit peut en outre comprendre une notice d'instructions comprenant des informations concernant l'administration d'une quantité efficace de chaque composé pour un sujet donné permettant de prévenir ou traiter une myopathie chez ce sujet.

Les figures et exemples suivants illustrent l'invention sans en limiter la portée.

### LEGENDES DES FIGURES

**Figure 1** **: Représentation schématique de la voie de signalisation de CD38.**
   L'ADP-ribosyl cyclase CD38 est une ecto enzyme qui produit à la fois I'ADP-ribose cyclique (cADPR) et le nicotinic acid adenine di-nucleotide phosphate (NAADP). Le cADPR active les récepteurs à la ryanodine (RYRs) localisés au niveau du réticulum sarcoplasmique (RE). Le NAADP active, *via* une protéine intermédiaire, un canal calcique localisé au niveau de la membrane du lysosome appelé le "two-pore channel" (TPC) mais aussi recrute directement ou indirectement les RyRs.
   Le "transient receptor potentiel cation channel 1" (TRPML1), membre de la sous-famille des mucolipines, peut être recruté indirectement par le NAADP. CICR ("Calcium-induced calcium release") désigne un mécanisme biologique où des ions calciums induisent la libération des ions calcium contenus dans le RE. GPCR: G Proteins/Coupled Receptors; EC: compartiment extracellulaire; IC: compartiment intracellulaire.

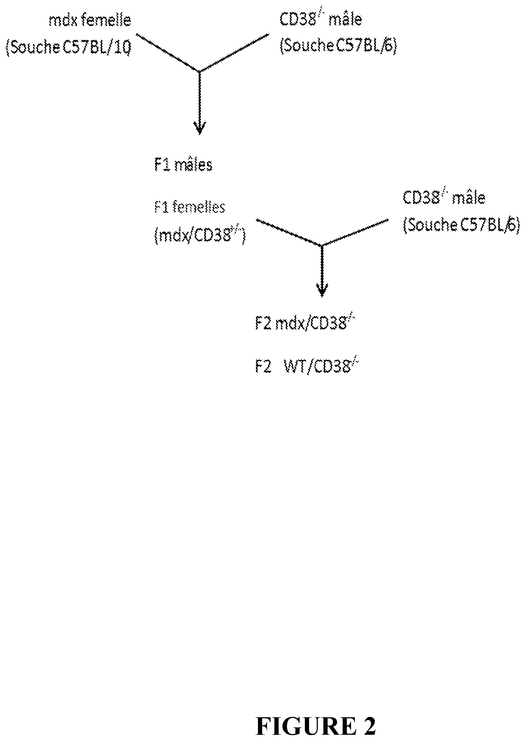

**Figure 2** **: Représentation des croisements pour obtenir des souris mdx/CD38^{-/-}.**

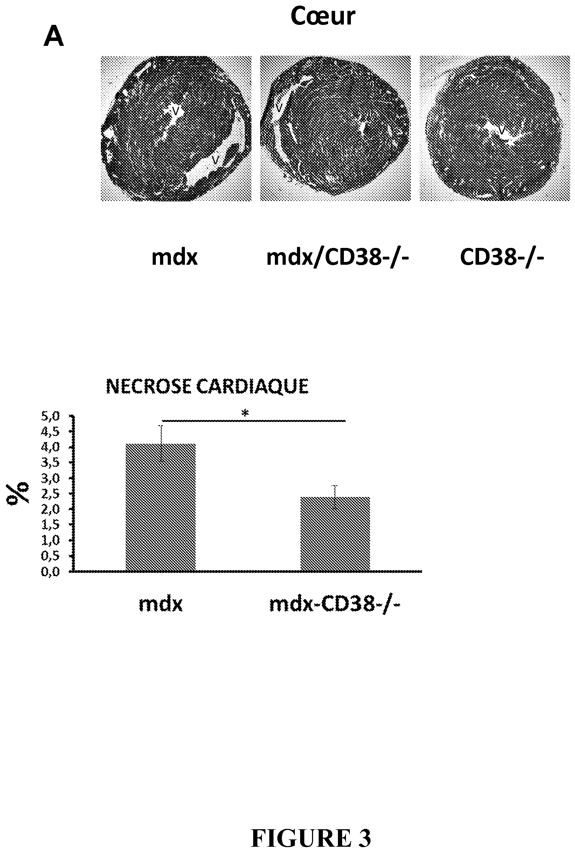

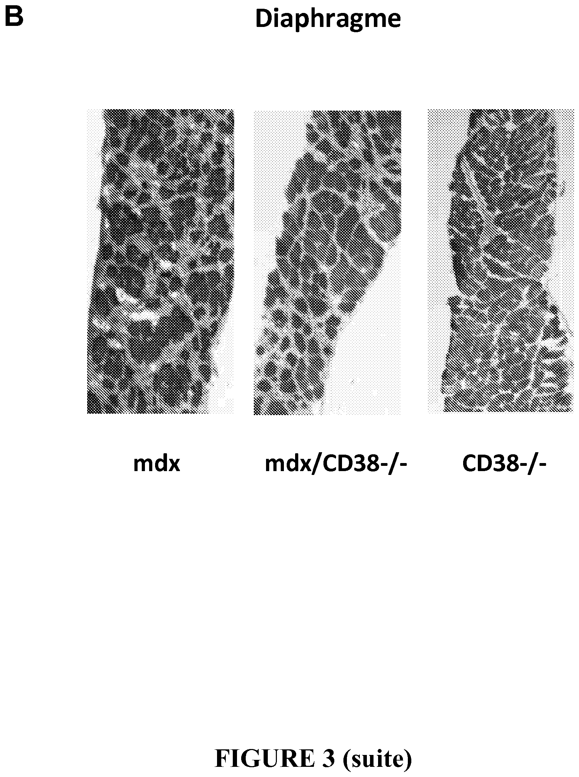

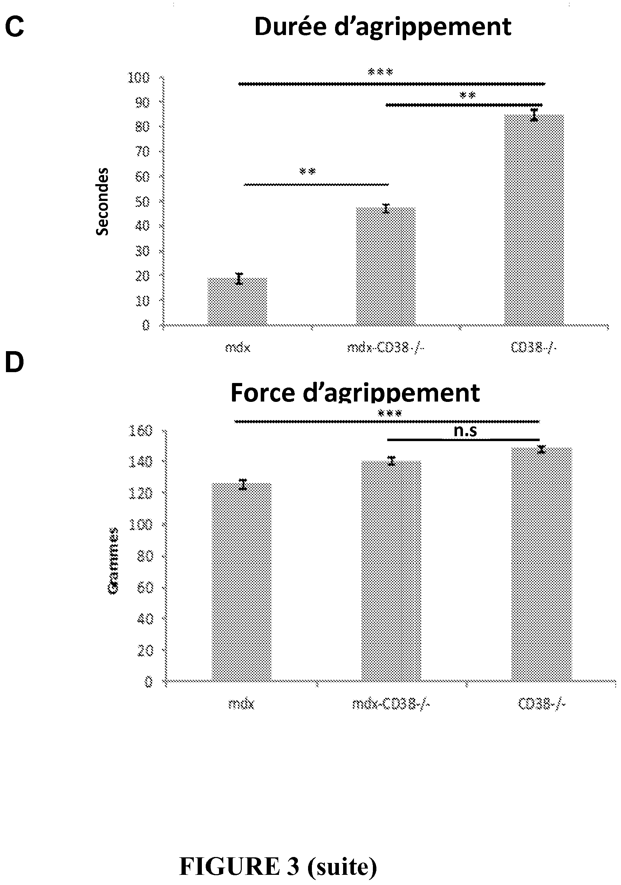

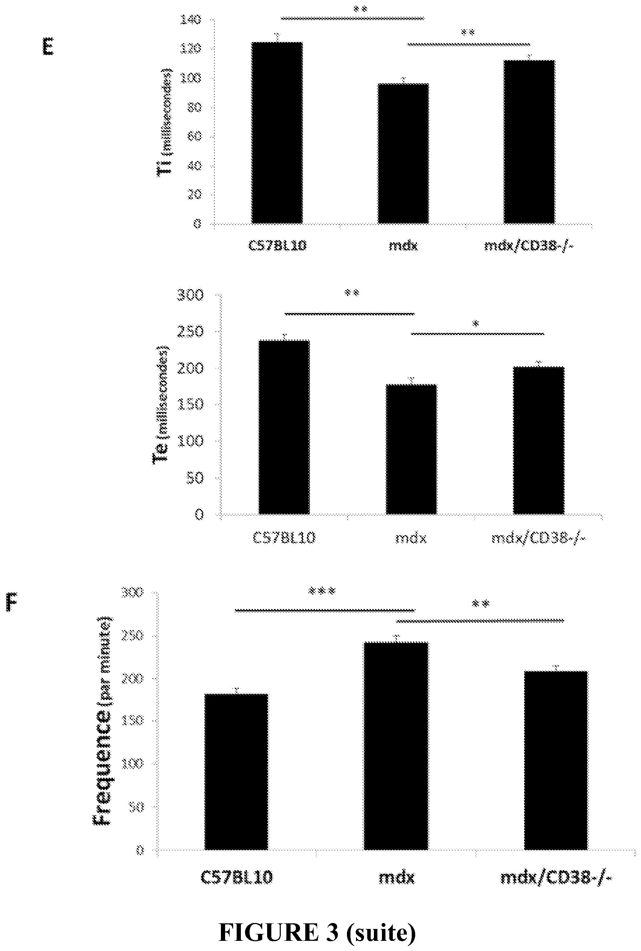

**Figure 3** **: (A-B) Bénéfice structural de l'invalidation du gène CD38 dans la souris *mdx.*** Amélioration de la structure du coeur et du diaphragme chez les souris *mdx* invalidées pour le gène CD38. La réduction de la nécrose dans le coeur (**A**) et le diaphragme (**B**) chez les souris *mdx*/CD38^{-/-}(n = 7) a été observée après coloration au trichrome de Masson, en comparant avec la nécrose observée chez la souris mdx (n = 13). L'absence de CD38 a réduit l'infiltration de collagène dans les tissus. Pourcentage de la surface de nécrose dans le coeur (coloration par le trichome de Masson) (A). Une réduction de 50% est observée chez les souris *mdx*/CD38^{-/-}(n = 7) comparées aux souris *mdx* (n = 13). La barre d'erreur représente ± SEM avec un seuil de significativité à *P≤ 0.05 (t-test). **(C-F) Bénéfice fonctionnel de l'invalidation du gène CD38 dans la souris *mdx.* (C) Test de résistance à la fatigue sur une grille inversée :** le temps de latence correspond au temps mis par la souris pour lâcher la grille. Plus le temps est long plus grande est la résistance à la fatigue. Ce temps est réduit de 78 % chez la souris *mdx* (n = 27) comparé à la souris CD38-/- (n = 45). L'invalidation du gène C38 chez la souris *mdx* (*mdx*/CD38^{-/-}, n = 33) améliore la performance de la souris *mdx* qui reste deux fois plus longtemps accrochée à la grille. **(D) Mesure de la force par le test d'agrippement:** la force d'agrippement est réduite de 15 % comparée aux souris CD38^{-/-} (n = 45). L'invalidation du gène CD38 dans la souris *mdx* (*mdx*/CD38^{-/-}, n = 33) améliore la force d'agrippement, la réduction n'étant plus que de 5% comparé à la souris CD38^{-/-}. La barre d'erreur représente ± SEM avec un seuil de significativité à *P≤ 0.05 (t-test) (n.s. = non significatif). (**E**, **F**) Mesure de la mécanique ventilatoire par pléthysmographie barométrique : (**E**) les temps inspiratoires et expiratoires (Ti (en haut) et Te (en bas)) sont réduits d'environ 25% chez la souris mdx et (**F**) la fréquence respiratoire est augmentée de 10%. Ces variations correspondent à des adaptations pour compenser la défaillance musculaire. L'invalidation du gène CD38 dans la souris mdx (mdx/CD38-/-, n = 9) améliore ces paramètres. La barre d'erreur représente ± SEM avec un seuil de significativité à *P≤ 0.05 (t-test et Mann-Whitney).

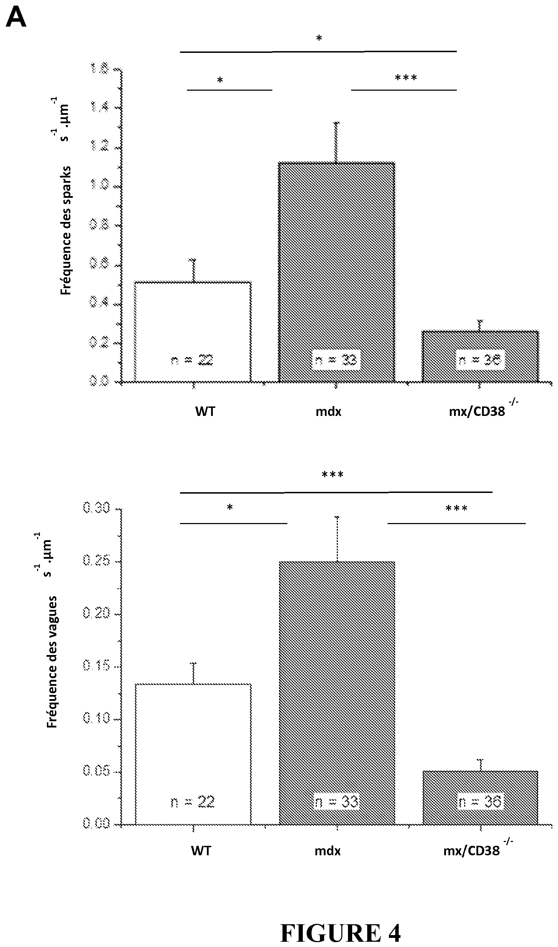

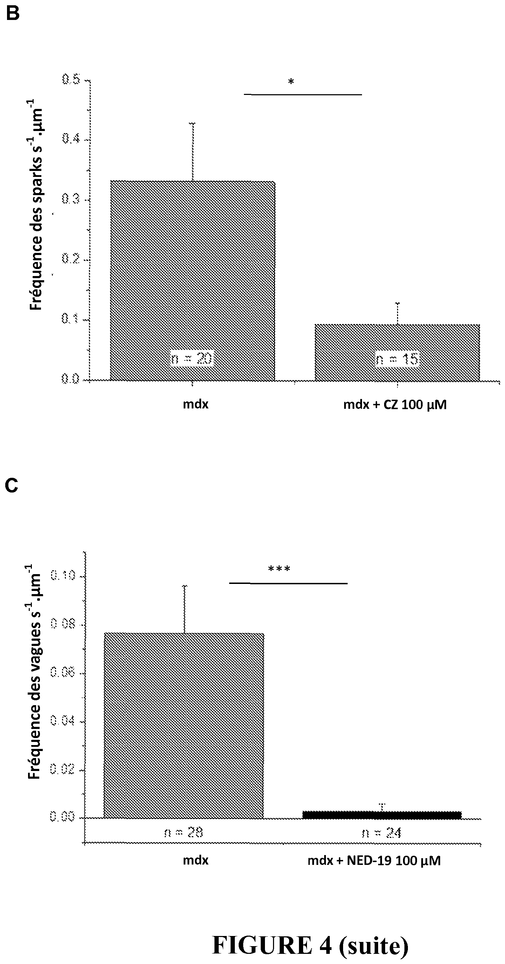

**Figure 4** **: Fréquence des sparks et vagues calciques spontanées dans les cardiomyocytes de souris WT, *mdx* and *mdx*/CD38^{-/-}.** 1-way-ANOVA test avec une correction de Bonferroni. Niveau de significativité fixé à 0,05, avec un *P < 0,05 ; **P < 0,01 , ***P < 0,001. (A) L'activité calcique spontanée des cardiomyocytes (sparks et vagues calciques) des souris mdx/CD38^{-/-} est réduite de 80% comparée aux valeurs des souris mdx. (**B**) L'application de l'antagoniste CZ-27 de CD38 sur des cardiomyocytes de souris mdx réduit d'environ 70 % la fréquence des sparks. (**C**) L'application de NED-19, un antagoniste du NAADP induit une réduction importante (environ 90 %) de l'activité des vagues calciques spontanées.

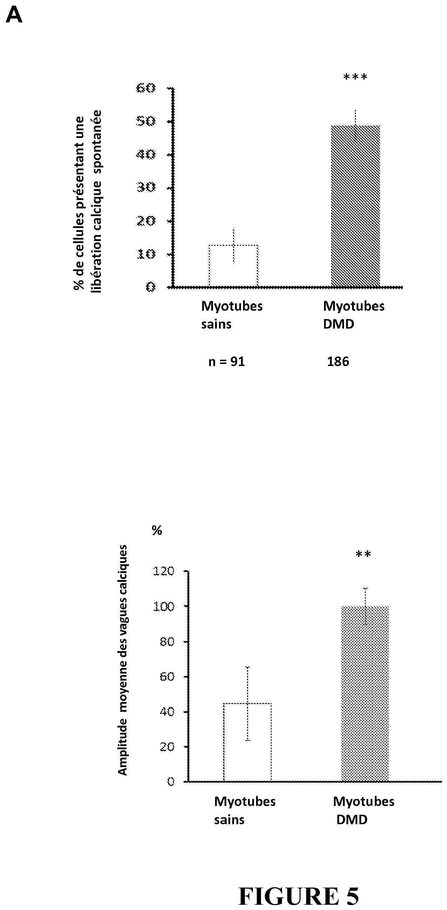

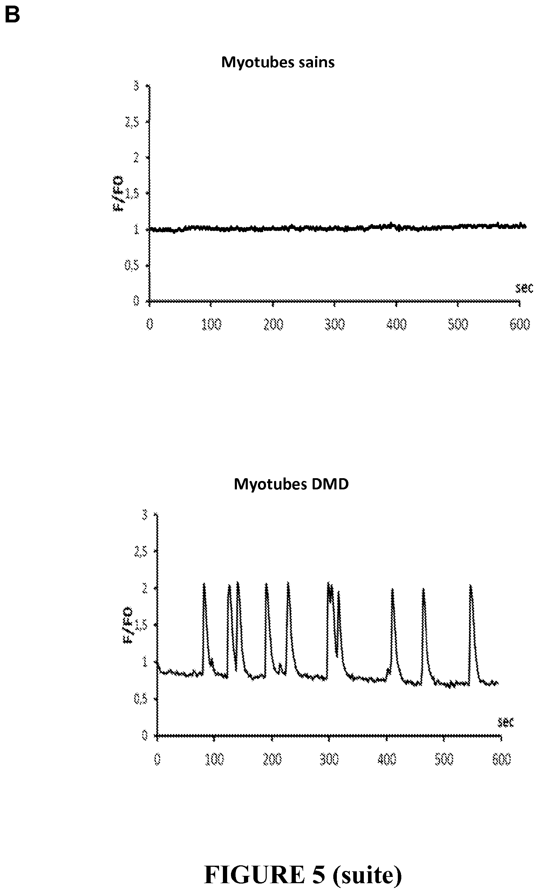

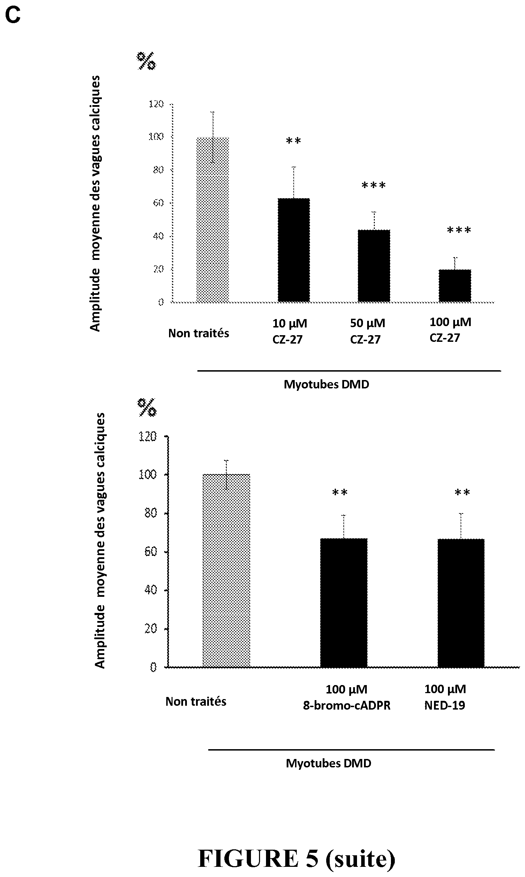

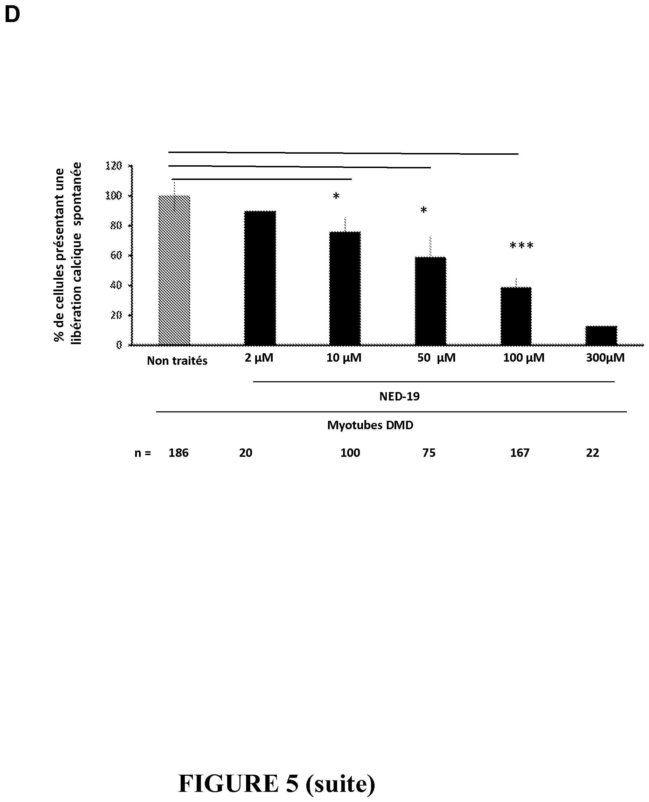

**Figure 5** **: Caractéristiques des activités calciques spontanées (vagues calciques) dans les myotubes DMD en absence ou en présence de modulateurs de la voie de l'enzyme CD38.** (**A**) Caractérisation des vagues calciques spontanées dans les myotubes DMD. Les myotubes sains présentent pour la plupart une faible activité (13% des myotubes), tandis que 49% des myotubes DMD non traités présentent une importante activité calcique spontanée. L'amplitude des oscillations calciques dans les myotubes DMD non traités est augmentée de 60 % comparé aux myotubes sains. (**B**) Exemples d'enregistrements de traces d'une région représentative d'intérêt dans des myotubes sains et DMD. (**C**) Amplitude des oscillations calciques spontanées dans les cultures de myotubes DMD en présence de modulateurs de la voie de l'enzyme CD38. Le CZ-27 (antagoniste du CD38) réduit fortement l'amplitude des oscillations calciques dans les myotubes DMD (n=23). De même, le 8-Bromo-cADPR (antagoniste du cADPR, 100µM, n=48) et le NED-19 (antagoniste du NAADP, 100µM, n=167) réduisent de 40 % l'amplitude des oscillations calciques dans les myotubes DMD. (D) Le pourcentage de cellules présentant une activité calcique spontanée est fortement réduit et de manière dose dépendante en présence de NED-19 (2-300 µM). Le t-test a été utilisé avec un niveau de significativité fixé à 0,05, avec un *P < 0,05 ; **P < 0,01 , ***P < 0,001, n= nombre de cellule.
**Figure 6** **: Le NED-19 est un antagoniste du NAADP** (ne fait pas partie de l'invention) [Rosen et al., J Biol Chem (2009) 284, 34930-34934 ; Davidson et al. Cardiovascular Research (2015) 108, 357-366].
**Figure 7** **: Représentation (A) de CZ-27 (antagoniste de CD38)** (Kwong et al., Biochemistry 2012, 51, 555-564) **et (B) du 8-Bromo-cADP-ribose (antagoniste du cADP-**ribose, ne fait pas partie de l'invention) (Walseth & Lee, Biochim. Biophys. Acta, 1178, 235 - 242 (1993).

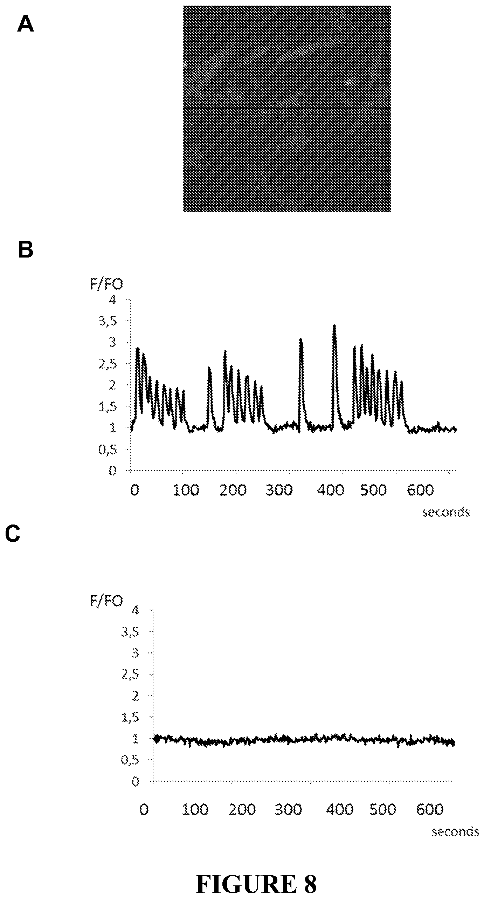

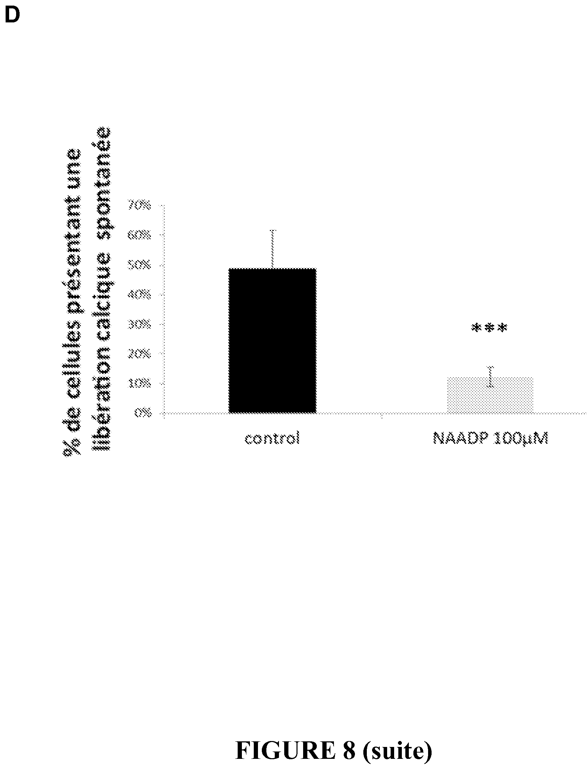

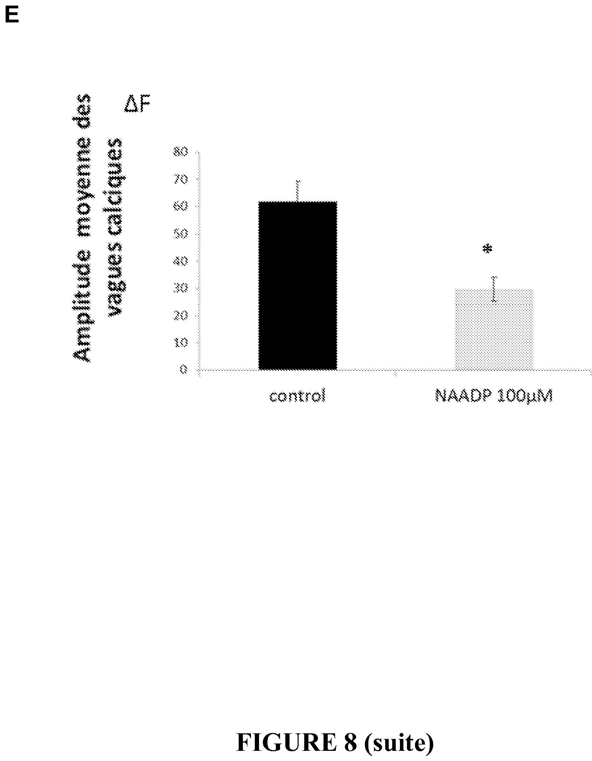

**Figure 8** **: Caractéristiques des activités calciques spontanées (vagues calciques) dans les myotubes DMD en absence ou en présence de NAADP, modulateur de la voie de l'enzyme CD38. (A)** Myotubes DMD chargés avec une sonde calcique (Fluo 4). Exemples d'enregistrements de traces d'une région représentative d'intérêt dans des myotubes DMD contrôles (**B**) et traités pendant 30 min avec du NAADP (100µM) (**C**). (**D**) le pourcentage de cellules présentant une activité calcique spontanée est fortement réduit en présence de NAADP 100µM. (**E**) l'amplitude des oscillations calciques spontanées est fortement réduite en présence de NAADP 100 µM. Le khideux (proportion de cellules actives) et le t-test (amplitude moyenne des pics) ont été utilisés avec un niveau de significativité fixé à 0,05, avec un *P < 0,05 ***P < 0,001.

### EXEMPLES

### Génération de souris Mdx/CD38^{-/-} :

Des souris déficientes en CD38 et des souris mdx sont utilisées. Ces souris ont été croisées afin d'obtenir des doubles-mutants (génération F2, Figure 2). Cinq génotypes de souris sont ainsi obtenus: les souris contrôles sont les C57BL/10 et les C57BL/6; les souris mutées sont les mdx, provenant de la souche des C57BL/10, et les CD38^{-/-}, provenant de la souche C57BL16 ; et enfin les souris mdx/CD38^{-/-} issues du croisement des souris femelles mdx et des souris mâles CD38^{-/-}. Leur contrôle correspondant est noté WT/CD38^{-/-} (cf. Figure 2). A partir des souris générées, seules les souris mâles ont été utilisées dans les expériences.

### Evaluation de la structure et de la fonction musculaire chez la souris :

Une évaluation phénotypique du double mutant a été réalisée. Pour cela, des animaux âgés de 4 à 12 mois ont été utilisés. Chez la souris mdx, la nécrose cardiaque se développe dès 4 mois et évolue peu jusqu'à 15 mois où elle devient plus importante alors que la nécrose du diaphragme est déjà très avancée dès l'âge de 4 mois. Dans le cas des souris mdx/CD38^{-/-}, une réduction très importante de la nécrose du diaphragme (cf. Figure 3B) et également une réduction de la nécrose cardiaque (cf. Figure 3A) associée à une amélioration fonctionnelle des muscles squelettiques (cf. Figure 3C, 3D) et du diaphragme (cf. Figure 3E, 3F) ont été observés.

### Evaluation de l'activité calcique spontanée du cardiomyocyte de souris :

Dans le cardiomyocyte mdx, il a été montré que les réserves calciques du réticulum sarcoplasmique étaient réduites, probablement en raison d'une fuite du Ca²⁺ via les RyRs qui sont anormalement sensibilisés. De plus, une augmentation de l'activité calcique spontanée de type spark a été observée chez les mdx.

Afin d'étudier l'impact de l'absence de CD38 sur ces paramètres, les différents paramètres de l'activité spontanée de type sparks et des vagues calciques ont été étudiés. Une augmentation de l'activité calcique spontanée (sparks et vagues calciques spontanés) liée à une sensibilité accrue des RyRs a été observée chez le cardiomyocyte de la souris mdx âgée de 4-5 mois (cf. Figure 4A). L'augmentation de la fréquence de ces évènements calciques spontanés peut conduire à des arythmies (lorsqu'ils se propagent à l'ensemble du tissu cardiaque) ainsi qu'à une hypertrophie cardiaque sur le long terme, liée à une modification de l'expression génique Ca²⁺-dépendante.

L'effet protecteur de la délétion de CD38 chez la souris a ensuite été observé en comparant les cardiomyocytes du modèle double-mutant aux cardiomyocytes des souris mdx. L'activité spontanée calcique des cardiomyocytes de mdx/CD38^{-/-} est très réduite comparée aux mdx (sparks et vagues calciques, cf. Figure 4A). Les résultats obtenus montrent une réduction de la sensibilité des RyRs chez la souris mdx/CD38^{-/-} (non illustré). Les résultats de la délétion de CD38 chez la mdx/CD38^{-/-} sont en faveur de son rôle protecteur dans la DMD, avec l'inversion de nombreux paramètres délétère chez la souris mdx (sparks et vagues calciques spontanés, sensibilisation des RyRs). L'application de l'antagoniste CZ-27 (cf. Figure 7A) de CD38 sur des cardiomyocytes de souris mdx améliore également certains paramètres comme une réduction de la fréquence des sparks (cf. Figure 4B). Lors que les cardiomyocytes de souris mdx sont incubées en présence NED-19 (cf. Figure 6), un antagoniste du NAADP, on observe une réduction importante du nombre de cardiomyocytes présentant de l'activité calcique spontanée (non illustré). Dans les rares cas d'activité spontanée en présence du NED-19, la fréquence des vagues calciques spontanée est fortement réduite (cf. Figure 4C).

### Effet du NED-19 sur l'activité calcique spontanée dans le myotube de patient atteint de la DMD (cf. Figure 5):

Parallèlement aux études réalisées sur les cardiomyocytes de souris, deux lignées de myotubes squelettiques humains (une lignée cellulaire de patient atteint de la DMD et une lignée contrôle) ont été utilisées. Les myotubes humains sont particulièrement adaptés pour évaluer un outil pharmacologique dans une perspective de thérapie. Les inventeurs ont dans un premier temps testé le CZ-27, le NED-19 antagoniste du NAADP (le messager le plus efficace produit par CD38, qui ne fait pas partie de l'invention), et le 8-Bromo-cADP-ribose (un antagoniste du cADP-ribose produit de CD38, qui ne fait pas partie de l'invention). Les cellules ont été chargées avec du Fluo-4 AM et enregistrées par microscopie confocale.

Seul 13 % des myotubes issus de la lignée saine contrôle ont présenté de l'activité calcique spontanée (vagues calciques) alors qu'environ 50% des myotubes issus de la lignée de patients atteint de la DMD ont présenté une activité calcique spontanée (cf. Figure 5A, B). L'amplitude des vagues calciques était réduite de 40 % (cf. Figure 5A) comparé à celle des myotubes DMD non traités. Lorsque les cultures de myotubes DMD ont été préincubées avec du CZ-27 (10, 50 et 100 µM), les inventeurs ont constaté que l'amplitude des vagues calciques était réduite de manière dose dépendante jusqu'à 80 % comparé à celle des myotubes DMD non traités (cf. Figure 5C). Une réduction de 40% de l'amplitude des vagues calciques est également observée lorsque les cellules sont traitées avec 100 µM de 8-Bromo-cADPR ou de NED-19 (cf. Figure 5C). Lorsque les cultures de myotubes DMD ont été préincubées avec du NED-19, le nombre de myotubes présentant une activité calcique spontanée a été réduit d'une manière dose-dépendante pour des concentrations de 2 à 300 µM (réduction de 60% à 100 µM) (cf. Figure 5D). De plus, quand les cultures de myotubes DMD ont été préincubées avec du NAADP à 100 µM, le nombre de myotubes présentant une activité calcique spontanée a été réduit (réduction de 75% avec 100 µM de NAADP) (cf. Figure 8D).

Les résultats obtenus par les inventeurs montrent que le NED-19 réduit très fortement l'occurrence et l'amplitude des vagues calciques spontanées suggérant que le NAADP est un élément central dans la genèse des signaux calciques anormaux dans cette lignée DMD. Le CZ-27 est lui le plus efficace pour réduire l'amplitude des vagues calciques spontanées dans les myotubes humains de patients DMD. Le 8 Bromo-cADPR a une activité sur l'amplitude des vagues calciques spontanées dans les myotubes humains de patient DMD équivalente à celle du NED-19 et peut être utilisé en synergie avec celui-ci.

### Conclusion :

L'inhibition de la voie de CD38 montre un bénéfice important sur les atteintes nécrotiques au niveau du coeur et du diaphragme, et sur l'atteinte fonctionnelle du muscle squelettique. Les résultats obtenus à la fois sur le cardiomyocyte et sur la lignée de myotube humain, démontrent que CD38 et ses métabolites (dont le NAADP) sont des cibles thérapeutiques clefs dans le traitement des myopathies, la DMD et la DMB en particulier, les composés NED-19 (qui ne fait pas partie de l'invention), 8-bromo-CADPR (qui ne fait pas partie de l'invention) et CZ-27 et leur dérivés étant des composés directement utilisables dans la prévention et le traitement desdites myopathies en ce qu'ils sont en mesure de normaliser les paramètres clefs de l'homéostasie calcique du muscle et de réduire la nécrose musculaire chez la souris mdx et dans une lignée cellulaire issue de patient atteint de DMD.

### REFERENCES

- Allen, D.G., Gervasio, O.L., Yeung, E.W., and Whitehead, N.P. (2010). Calcium and the damage pathways in muscular dystrophy. Can. J. Physiol. Pharmacol. 88, 83- 91.
- Altamirano, F., López, J.R., Henríquez, C., Molinski, T., Allen, P.D., and Jaimovich, E. (2012). Increased resting intracellular calcium modulâtes NF-κB-dependent inducible nitric-oxide synthase gène expression in dystrophie mdx skeletal myotubes. J. Biol. Chem. 287, 20876-20887.
- Au, C.G., Butler, T.L., Sherwood, M.C., Egan, J.R., North, K.N., and Winlaw, D.S. (2011). Increased connective tissue growth factor associated with cardiac fibrosis in the mdx mouse model of dystrophie cardiomyopathy. Int. J. Exp. Pathol. 92, 57- 65.
- Basset, O., Boittin, F.-X., Cognard, C., Constantin, B., and Ruegg, U.T. (2006). Bcl-2 overexpression prevents calcium overload and subséquent apoptosis in dystrophie myotubes. Biochem. J. 395, 267- 276.
- Capel RA, Bolton EL, Lin WK, Aston D, Wang Y, Liu W, Wang X, Burton RB, Bloor-Young D, Shade KT, Ruas M, Parrington J, Churchill GC, Lei M, Galione A, Terrar DA. (2015) Two pore channels (TPC2s) and nicotinic acid adenine dinucleotide phosphate (NAADP) at lysosomal-sarcoplasmic reticular junctions contribute to acute and chronic β-adrenoceptor signaling in the heart. J Biol Chem. 2015 Oct 5. pii: jbc.M115.684076. [Epub ahead of print]
- Cosker, F., Cheviron, N., Yamasaki, M., Menteyne, A., Lund, F.E., Moutin, M.-J., Galione, A., and Cancela, J.-M. (2010). The ecto-enzyme CD38 is a nicotinic acid adenine dinucleotide phosphate (NAADP) synthase that couples receptor activation to Ca2+ mobilization from lysosomes in pancreatic acinar cells. J. Biol. Chem. 285, 38251-38259.
- Davidson SM, Foote K, Kunuthur S, Gosain R, Tan N, Tyser R, Zhao YJ, Graeff R, Ganesan A, Duchen MR, Patel S, Yellon DM. (2015) Inhibition of NAADP signalling on reperfusion protects the heart by preventing lethal calcium oscillations via two-pore channel 1 and opening of the mitochondrial permeability transition pore. Cardiovasc Res. Dec 1;108(3):357-66.
- Galione, A. (2015). A primer of NAADP-mediated Ca(2+) signalling: From sea urchin eggs to mammalian cells. Cell Calcium. Jul; 58 (1):27-47.
- Kwong, A.K.Y., Chen, Z., Zhang, H., Leung, F.P., Lam, C.M.C., Ting, K.Y., Zhang, L., Hao, Q., Zhang, L.-H., and Lee, H.C. (2012). Catalysis-based inhibitors of the calcium signaling function of CD38. Biochemistry (Mosc.) 51, 555-564.
- Lewis, A.M., Aley, P.K., Roomi, A., Thomas, J.M., Masgrau, R., Garnham, C., Shipman, K., Paramore, C., Bloor- Young, D., Sanders, L.E.L., et al. (2012). β-Adrenergic receptor signaling increases NAADP and cADPR levels in the heart. Biochem. Biophys. Res. Commun.427, 326-329.
- Naylor, E., Arredouani, A., Vasudevan, S.R., Lewis, A.M., Parkesh. R., Mizote, A., Rosen, D., Thomas, J.M., Izumi, M., Ganesan, A., et al. (2009). Identification of a chemical probe for NAADP by virtual screening. Nat. Chem. Biol. 5, 220-226.
- Nebel, M., Schwoerer, A.P., Warszta, D., Siebrands, C.C., Limbrock, A.-C., Swarbrick, J.M., Fliegert, R., Weber, K., Bruhn, S., Hohenegger, M., et al. (2013). Nicotinic acid adenine dinucleotide phosphate (NAADP)-mediated calcium signaling and arrhythmias in the heart evoked by β-adrenergic stimulation. J. Biol. Chem. 288, 16017-16030.
- Partida-Sánchez, S., Cockayne, D.A., Monard, S., Jacobson, E.L., Oppenheimer, N., Garvy, B., Kusser, K., Goodrich, S., Howard, M., Harmsen, A., et al. (2001). Cyclic ADP-ribose production by CD38 regulates intracellular calcium release, extracellular calcium influx and chemotaxis in neutrophils and is required for bacterial clearance in vivo, Nat. Med. 7, 1209-1216.
- Quinlan, J.G., Hahn, H.S., Wong, B.L., Lorenz, J.N., Wenisch, A.S., and Levin, L.S. (2004). Evolution of the mdx mouse cardiomyopathy: physiological and morphological findings. Neuromuscul Disord 14,491-496.
- Rosen et al., J Biol Chem (2009) 284, 34930-34934
- Sabourin, J., Harisseh, R., Harnois, T., Magaud, C., Bourmeyster, N., Déliot, N., and Constantin, B. (2012). Dystrophin/α1-syntrophin scaffold regulated PLC/PKC-dependent store-operated calcium entry in myotubes. Cell Calcium.
- Sarma, S., Li, N., van Oort, R.J., Reynolds, C., Skapura, D.G., and Wehrens, X.H.T. (2010). Genetic inhibition of PKA phosphorylation of RyR2 prevents dystrophie cardiomyopathy. Proc. Natl. Acad. Sci. U.S.A. 107, 13165-13170.
- Sethi et al,. J Biol Chem. 1997. Vol. 272, No. 26, pp. 16358-16363
- Spumey, C.F., Knoblach, S., Pistilli, E.E., Nagaraju, K., Martin, G.R., and Hoffman, E.P. (2008). Dystrophin-deficient cardiomyopathy in mouse: expression ofNox4 and Lox are associated with fibrosis and altered functional parameters in the heart. Neuromuscul. Disord. NMD 18,371-381.
- Takahashi, J., Kagaya, Y., Kato, I., Ohta, J., Isoyama, S., Miura, M., Sugai, Y., Hirose, M., Wakayama, Y., Ninomiya, M., et al. (2003). Déficit of CD38/cyc1ic ADP-ribose is differentially compensated in hearts by gender. Biochem. Biophys. Res. Commun. 312, 434-440.
- Vianello, S., Consolaro, F., Bich, C., Cancela, J.-M., Roulot, M., Lanchec, E., Touboul, D., Brunelle, A., Israël, M., Benoit, E., et al. (2014a). Low doses of arginine butyrate dérivatives improve dystrophie phenotype and restore membrane integrity in DMD models. FASEB J. Off. Publ. Fed. Am. Soc. Exp. Biol.; 28(6):2603-2619.
- Vianello, S., Bouyon, S., Benoit, E., Sebrié, C., Boerio, D., Herbin, M., Roulot, M., Fromes, Y., de la Porte, S. (2014). Arginine butyrate per os protects mdx mice against cardiomyopathy, kyphosis and changes in axonal excitability. Neurobiol Dis.; 71: 325-333.
- Walseth, T.F., and Lee, H.C. (1993). Synthesis and characterization of antagonists of cyclic-ADP-ribose-induced Ca2+ release. Biochim. Biophys. Acta 1178,235-242.
- Wang, S., Zhu, W., Wang, X., Li, J., Zhang, K., Zhang, L., Zhao, Y.-J., Lee, H.C., and Zhang, L. (2014). Design, synthesis and SAR studies of NAD analogues as potent inhibitors towards CD38 NADase. Mol. Basel Switz. 19, 15754-15767.
- Wei, W.-J., Sun, H.-Y., Ting, K.Y., Zhang, L.-H., Lee, H.-C., Li, G.-R., and Yue, J. (2012). Inhibition of cardiomyocytes différentiation of mouse embryonic stem cells by CD38/cADPR/Ca2+ signaling pathway. J. Biol. Chem.287, 35599-35611.

## Revendications

1. Composé antagoniste des ADP ribosyl cyclases ou antagoniste de l'acide nicotinique adénine dinucléotide phosphate (NAADP) pour une utilisation pour prévenir ou traiter une myopathie ou pour traiter un traumatisme musculaire chez un sujet, la myopathie n'étant pas une myopathie urémique, **caractérisé en ce que** l'ADP ribosyl cyclase est CD38 et **en ce que** le composé antagoniste du CD38 est un composé de formule (II) : 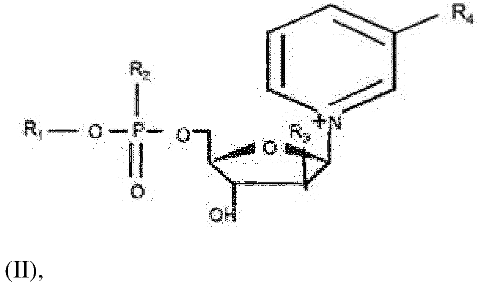 dans laquelle:
- R₁ = H, C₃H₇, C₄H₉, C₆H₁₃, C₁₂H₂₅, groupe CH₂-benzene ou CH₂-CH₂-Benzene ;
- R₂ = S, OH,
- R₃ = F, CN, Br, Cl et/ou
- R₄ = CONH₂, COOH, COCN, ou COF ;
Et l'antagoniste de l'acide nicotinique adénine dinucléotide phosphate (NAADP) est le NAADP de formule : 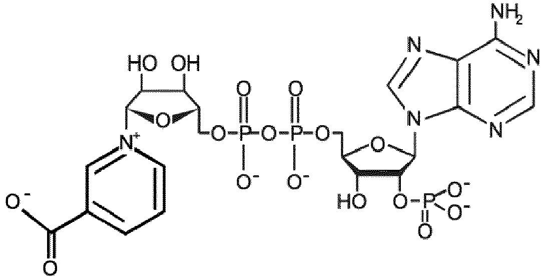
à une concentration d'au moins 100 µmolaires ;
et/ou mélange d'au moins deux desdits composés.

2. Composé ou mélange de composés pour leur utilisation selon la revendication 1, **caractérisé en ce que** la myopathie est sélectionnée parmi une dystrophie musculaire et une cardiomyopathie.

3. Composé ou mélange de composés pour leur utilisation selon la revendication 2, **caractérisé en ce que** la dystrophie musculaire est sélectionnée parmi la dystrophie musculaire de Duchenne (DMD) et la dystrophie musculaire de Becker (DMB).

4. Composé pour son utilisation selon la revendication 1, **caractérisé en ce que** le composé antagoniste du CD38 est le composé CZ-27 dans lequel R₁ = C₄H₉, R₂ = OH, R₃ = F et R₄ = CONH₂, le composé CZ-48 dans lequel R₁ = H, R₂ = S, R₃ = F et R₄ = CONH₂.

5. Composé ou mélange de composés selon l'une quelconque des revendications 1 ou 4, pour une utilisation pour prévenir ou traiter au moins un symptôme de la dystrophie musculaire sélectionné parmi une dégénérescence musculaire, une faiblesse musculaire (hypotonicité), une myotonie, une insuffisance cardiaque d'origine musculaire, une insuffisance respiratoire d'origine musculaire, une inflammation et un retard mental.

6. Composé ou mélange de composés selon l'une quelconque des revendications 1 ou 4, pour une utilisation pour prévenir ou traiter la dystrophie musculaire en combinaison avec un composé distinct utilisé classiquement dans la prévention ou le traitement de ladite dystrophie, ledit composé distinct étant de préférence sélectionné parmi un glucorticoïde tel que la prednisolone ou le deflazacort, un bétabloquant tel que l'albutérol, la diphénylhydantoïne (Dilantin^{®}), la méxilétine (Mexitil^{®}), le baclofène (Liorésal@), le dantrolène (Dantrium^{®}) ou la carbamazépine (Tégrétol^{®}), et un inhibiteur de l'enzyme de conversion de l'angiotensine (ECA).

7. Composé ou mélange de composés selon l'une quelconque des revendications 1 ou 4, pour une utilisation pour prévenir ou traiter la cardiomyopathie en combinaison avec un composé distinct utilisé classiquement dans la prévention ou le traitement des cardiomyopathies, ledit composé distinct étant de préférence sélectionné parmi l'acébutolol (Sectral^{®}), l'albutérol, la diphénylhydantoïne (Dilantin^{®}), la méxilétine (Mexitil^{®}), le baclofène (Liorésal@), le dantrolène (Dantrium^{®}) et la carbamazépine (Tégrétol^{®}).

8. Composé ou mélange de composés selon l'une quelconque des revendications 1 ou 4, pour une utilisation pour traiter le traumatisme musculaire en combinaison avec un composé distinct utilisé classiquement dans le traitement des traumatismes musculaires, ledit composé distinct étant de préférence sélectionné parmi un tonique veineux, un anti-oedémateux et un anti-inflammatoire.

9. Composition pharmaceutique pour utilisation pour prévenir ou traiter une myopathie ou pour traiter un traumatisme musculaire chez un sujet, la myopathie n'étant pas une myopathie urémique comprenant un composé selon l'une quelconque des revendications 1 ou 4 et un support acceptable sur le plan pharmaceutique, tel que le composé n'est pas le NAADP.

10. Kit prophylactique ou thérapeutique comprenant composé selon l'une quelconque des revendications 1 ou 4, et/ou une composition selon la revendication 9, et au moins un composé sélectionné parmi un glucorticoïde tel que la prednizolone ou le deflazacort, un bétabloquant tel que l'albutérol, la diphénylhydantoïne (Dilantin^{®}), la méxilétine (Mexitil@), le baclofène (Liorésal@), le dantrolène (Dantrium^{®}) ou la carbamazépine (Tégrétol^{®}), un inhibiteur de l'enzyme de conversion de l'angiotensine (ECA), et une séquence nucléotidique permettant de modifier l'expression de la dystrophine, lesdits composés se présentant de préférence dans des containers distincts.

## Patentansprüche

1. ADP-Ribosyl-Cyclase-Antagonist oder Nikotinsäure-Adenin-Dinukleotid-Phosphat (NAADP)-Antagonist-Verbindung zur Verwendung bei der Vorbeugung oder Behandlung von Myopathie oder der Behandlung von Muskeltrauma bei einem Patienten, wobei die Myopathie keine urämische Myopathie ist, **dadurch gekennzeichnet, dass** die ADP-Ribosyl-Cyclase CD38 ist und dass die CD38-Antagonist-Verbindung eine Verbindung der Formel (II) ist: 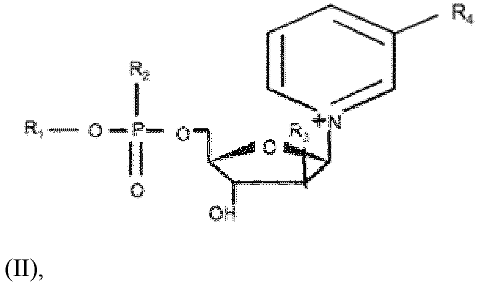 in denen:
- R₁ = H, C₃H₇, C₄H₉ , C₆H₁₃ , C₁₂H₂₅ , CH₂ -Benzol oder CH₂-CH₂-Benzolgruppe;
- R₂= S, OH,
- R₃ = F, CN, Br, Cl und/oder
- R₄ = CONH₂ , COOH, COCN, oder COF ;
und der Nikotinsäure-Adenin-Dinukleotid-Phosphat (NAADP)-Antagonist ist NAADP der Formel: 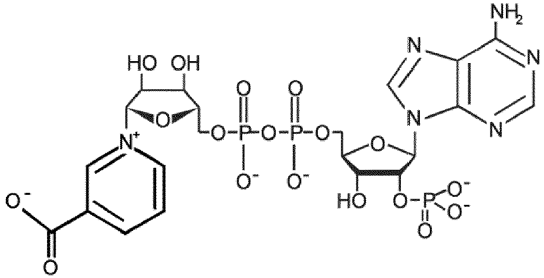
in einer Konzentration von mindestens 100 µmolar;
und/oder ein Gemisch aus mindestens zwei der genannten Verbindungen.

2. Verbindung oder Mischung von Verbindungen zur Verwendung gemäß Anspruch 1, **dadurch gekennzeichnet, dass** die Myopathie ausgewählt ist aus Muskeldystrophie und Kardiomyopathie.

3. Verbindung oder Mischung von Verbindungen zur Verwendung gemäß Anspruch 2, **dadurch gekennzeichnet, dass** die Muskeldystrophie ausgewählt ist aus Duchenne-Muskeldystrophie (DMD) und Becker-Muskeldystrophie (BMD).

4. Verbindung zur Verwendung gemäß Anspruch 1, **dadurch gekennzeichnet, dass** die CD38-Antagonistenverbindung die Verbindung CZ-27 ist, in der R₁ = C₄H₉, R₂ = OH, R₃ = F und R₄ = CONH₂ , die Verbindung CZ-48, in der R₁ = H, R₂ = S, R₃ = F und R₄ = CONH₂ .

5. Verbindung oder Mischung von Verbindungen nach einem der Ansprüche 1 oder 4 zur Verwendung bei der Vorbeugung oder Behandlung von mindestens einem Symptom der Muskeldystrophie, ausgewählt aus Muskeldegeneration, Muskelschwäche (Hypotonie), Myotonie, muskulärem Herzversagen, muskulärem Atemversagen, Entzündung und geistiger Retardierung.

6. Verbindung oder Mischung von Verbindungen nach einem der Ansprüche 1 oder 4 zur Verwendung bei der Vorbeugung oder Behandlung von Muskeldystrophie in Kombination mit einer getrennten Verbindung, die üblicherweise bei der Vorbeugung oder Behandlung der Dystrophie verwendet wird, wobei die getrennte Verbindung vorzugsweise aus einem Glucorticoid wie Prednisolon oder Deflazacort, einem Betablocker wie Albuterol, Diphenylhydantoin (Dilantin^{®}), Mexiletin (Mexitil^{®}), Baclofen (Liorésal^{®}), Dantrolen (Dantrium^{®}) oder Carbamazepin (Tégrétol^{®}) und einem Angiotensin-konvertierenden Enzym (ACE)-Inhibitor ausgewählt ist.

7. Verbindung oder Mischung von Verbindungen nach einem der Ansprüche 1 oder 4 zur Verwendung bei der Vorbeugung oder Behandlung von Kardiomyopathie in Kombination mit einer getrennten Verbindung, die üblicherweise bei der Vorbeugung oder Behandlung von Kardiomyopathie verwendet wird, wobei die getrennte Verbindung vorzugsweise ausgewählt ist aus Acebutolol (Sectral^{®}), Albuterol, Diphenylhydantoin (Dilantin^{®}), Mexiletin (Mexitil^{®}), Baclofen (Liorésal^{®}), Dantrolen (Dantrium^{®}) und Carbamazepin (Tégrétol^{®}).

8. Verbindung oder Mischung von Verbindungen nach einem der Ansprüche 1 oder 4 zur Verwendung bei der Behandlung von Muskeltrauma in Kombination mit einer separaten Verbindung, die üblicherweise bei der Behandlung von Muskeltrauma verwendet wird, wobei die separate Verbindung vorzugsweise aus einem Venentonikum, einem Anti-Ödematösem und einem Entzündungshemmer ausgewählt ist.

9. Pharmazeutische Zusammensetzung zur Verwendung bei der Vorbeugung oder Behandlung von Myopathie oder der Behandlung von Muskeltrauma in einem Subjekt, wobei die Myopathie keine urämische Myopathie ist, umfassend eine Verbindung nach einem der Ansprüche 1 oder 4 und einen pharmazeutisch akzeptablen Träger, so dass die Verbindung nicht NAADP ist.

10. Prophylaktisches oder therapeutisches Kit, umfassend eine Verbindung nach einem der Ansprüche 1 oder 4 und/oder eine Zusammensetzung nach Anspruch 9 und mindestens eine Verbindung, ausgewählt aus einem Glucorticoid wie Prednizolon oder Deflazacort, einem Betablocker wie Albuterol, Diphenylhydantoin (Dilantin^{®}), Mexiletin (Mexitil^{®}), Baclofen (Liorésal^{®}), Dantrolen (Dantrium^{®}) oder Carbamazepin (Tégrétol^{®}), einem Inhibitor des Angiotensin-konvertierenden Enzyms (ACE) und einer Nukleotidsequenz, die es ermöglicht, die Expression von Dystrophin zu modifizieren, ausgewählt ist, wobei diese Verbindungen vorzugsweise in getrennten Behältnissen vorliegen.

## Claims

1. An ADP ribosyl cyclase antagonist or nicotinic acid adenine dinucleotide phosphate (NAADP) antagonist compound for use in preventing or treating myopathy or treating muscle trauma in a subject, the myopathy not being uraemic myopathy, **characterised in that** the ADP ribosyl cyclase is CD38 and **in that** the CD38 antagonist compound is a compound of formula (II) : 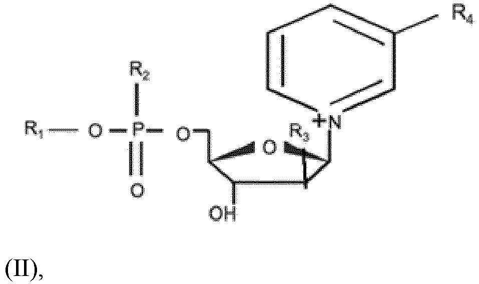 in which:
- R₁ = H, C₃H₇, C₄H₉ , C₆H₁₃ , C₁₂H₂₅ , CH₂-benzene or CH₂-CH₂-Benzene group ;
- R₂ = S, OH,
- R₃ = F, CN, Br, Cl and/or
- R₄ = CONH₂ , COOH, COCN, or COF ;
and the nicotinic acid adenine dinucleotide phosphate (NAADP) antagonist compound is NAADP of formula : 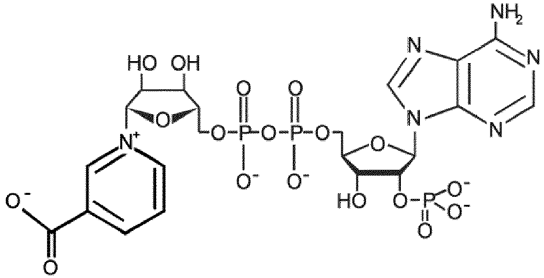
at a concentration of at least 100 µmolar ;
and/or a mixture of at least two of the said compounds.

2. A compound or mixture of compounds for use according to claim 1, **characterised in that** the myopathy is selected from muscular dystrophy and cardiomyopathy.

3. A compound or mixture of compounds for use according to claim 2, **characterised in that** the muscular dystrophy is selected from Duchenne muscular dystrophy (DMD) and Becker muscular dystrophy (BMD).

4. A compound for use according to claim 1, **characterised in that** the CD38 antagonist compound is the compound CZ-27 in which R₁ = C₄H₉, R₂ = OH, R₃ = F and R₄ = CONH₂ , the compound CZ-48 in which R₁ = H, R₂ = S, R₃ = F and R₄ = CONH₂ .

5. A compound or mixture of compounds according to any one of claims 1 or 4, for use in preventing or treating at least one symptom of muscular dystrophy selected from muscular degeneration, muscular weakness (hypotonicity), myotonia, muscular heart failure, muscular respiratory failure, inflammation and mental retardation.

6. A compound or mixture of compounds according to any one of claims 1 or 4, for use in preventing or treating muscular dystrophy in combination with a separate compound conventionally used in the prevention or treatment of said dystrophy, said distinct compound preferably being selected from a glucorticoid such as prednisolone or deflazacort, a beta-blocker such as albuterol, diphenylhydantoin (Dilantin^{®}), mexiletine (Mexitil^{®}), baclofen (Liorésal^{®}), dantrolene (Dantrium^{®}) or carbamazepine (Tégrétol^{®}), and an angiotensin-converting enzyme (ACE) inhibitor.

7. A compound or mixture of compounds according to any one of claims 1 or 4, for use in preventing or treating cardiomyopathy in combination with a separate compound conventionally used in the prevention or treatment of cardiomyopathy, said distinct compound preferably being selected from acebutolol (Sectral^{®}), albuterol, diphenylhydantoin (Dilantin^{®}), mexiletin (Mexitil^{®}), baclofen (Liorésal^{®}), dantrolene (Dantrium^{®}) and carbamazepine (Tégrétol^{®}).

8. A compound or mixture of compounds according to any one of claims 1 or 4, for use in treating muscle trauma in combination with a separate compound conventionally used in the treatment of muscle trauma, said separate compound preferably being selected from a venous tonic, an anti-oedematous and an anti-inflammatory.

9. A pharmaceutical composition for use in preventing or treating myopathy or treating muscle trauma in a subject, the myopathy not being uraemic myopathy comprising a compound of any one of claims 1 or 4 and a pharmaceutically acceptable carrier, such that the compound is not NAADP.

10. A prophylactic or therapeutic kit comprising a compound according to any one of claims 1 or 4, and/or a composition according to claim 9, and at least one compound selected from a glucorticoid such as prednizolone or deflazacort, a beta-blocker such as albuterol, diphenylhydantoin (Dilantin^{®}), mexiletin (Mexitil^{®}), baclofen (Liorésal^{®}), dantrolene (Dantrium^{®}) or carbamazepine (Tégrétol^{®}), an angiotensin-converting enzyme (ACE) inhibitor, and a nucleotide sequence making it possible to modify the expression of dystrophin, the said compounds preferably being in separate containers.
